(19) Europäisches Patentamt
European Patent Office
Office européen des brevets

(11) Numéro de publication: **0 288 394 B1**

(12) **FASCICULE DE BREVET EUROPEEN**

(45) Date de publication de fascicule du brevet: **09.09.92**

(51) Int. Cl.⁵: **C07D 211/70**, C07D 211/94, A61K 31/445

(21) Numéro de dépôt: **88400996.0**

(22) Date de dépôt: **22.04.88**

(54) **Dérivés de la 1,2,5,6-tétrahydropyridine, leur procédé de préparation, leur application comme médicaments et les compositions les renfermant.**

(30) Priorité: **24.04.87 IT 2026087**

(43) Date de publication de la demande:
**26.10.88 Bulletin 88/43**

(45) Mention de la délivrance du brevet:
**09.09.92 Bulletin 92/37**

(84) Etats contractants désignés:
**AT BE CH DE ES FR GB GR IT LI LU NL SE**

(56) Documents cités:
**FR-A- 1 258 847**
**US-A- 4 710 508**

**JOURNAL OF PHARMACEUTICAL SCIENCES, vol. 56, n 9, septembre 1967, pages 1190-1192; J.N. WELLS et al.: "Synthesis of aldoxime analogs of arecoline as reactivators of organophosphorus inhibited cholinesterase"**

(73) Titulaire: **ROUSSEL-UCLAF**
**35, Boulevard des Invalides**
**F-75007 Paris(FR)**

(72) Inventeur: **Galliani, Giulio**
**13, Via Silva**
**1 Monza (MI)(IT)**
Inventeur: **Barzaghi, Fernando**
**Via Monteverdi 21**
**I-20052 Monza (MI)(IT)**
Inventeur: **Bonetti, Carla**
**Via Beccalino**
**Fontanella (Bergamo)(IT)**
Inventeur: **Toja, Emilio**
**Via Plezzo 80**
**Milano(IT)**

(74) Mandataire: **Tonnellier, Marie-José et al**
**111, route de Noisy B.P. no 9**
**F-93230 Romainville(FR)**

EP 0 288 394 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EUROPEAN JOURNAL OF MEDICINE CHEMISTRY - CHIMICA THERAPEUTICA, vol. 9, n 1, janvier/février 1974, pages 101-107; P. VERGNON et al.: "Dérivés vinylcyclopropaniques II. - Pharmacologie de l'oxime de l'acétyl-1 vinyl-2 cyclopropane"

CHEMICAL ABSTRACTS, vol. 90, n 21, 21 mai 1979, page 585, résumé no. 168416x, Columbus, Ohio, US; E.K. ORLOVA et al.: "Synthesis and neurotropic properties of some alpha-aminobenzyl piperidines"

## Description

L'invention concerne de nouveaux dérivés de la 1,2,5,6-tétrahydropyridine et leurs sels, leur procédé de préparation, leur application comme médicaments et les compositions les renfermant.

L'invention a pour objet les composés de formule (I) :

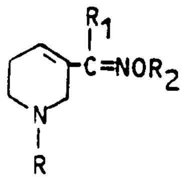

(I)

dans laquelle R représente un atome d'hydrogène, un radical hydroxyle, un radical alkyle linéaire, ramifié ou cyclique, saturé ou insaturé renfermant jusqu'à 8 atomes de carbone, ou R représente un radical aralkyle renfermant jusqu'à 10 atomes de carbone, $R_1$ représente un radical alkyle linéaire, ramifié ou cyclique, saturé ou insaturé renfermant jusqu'à 8 atomes de carbone et $R_2$ représente un atome d'hydrogène ou un radical alkyle linéaire ou ramifié, saturé ou insaturé renfermant jusqu'à 8 atomes de carbone, un radical $COalc_1$ ou un radical $(CH_2)_2N(alc_2)_2$, $alc_1$ et $alc_2$ représentant un radical alkyle renfermant jusqu'à 8 atomes de carbone ainsi que leurs sels d'addition avec les acides à la condition que si R représente un radical alkyle, $R_2$ ne représente pas un atome d'hydrogène.

Parmi les sels d'addition avec les acides, on peut citer ceux formés avec les acides minéraux, tels que les acides chlorhydrique, bromhydrique, sulfurique ou phosphorique ou avec les acides organiques comme l'acide formique, acétique, propionique, benzoïque, maléique, fumarique, succinique, tartrique, citrique, oxalique, glyoxylique, aspartique, alcane-sulfoniques, tels que les acides méthane ou éthane sulfoniques, arylsulfoniques tels que les acides benzène ou para-toluènesulfoniques.

Lorsque R, $R_1$ ou $R_2$ représente un radical alkyle saturé, linéaire ou ramifié, il s'agit de préférence d'un radical méthyle, éthyle, n-propyle, isopropyle, n-butyle, sec-butyle, isobutyle, n-pentyle, n-hexyle.

Lorsque R, $R_1$ ou $R_2$ représente un radical alkyle insaturé, il s'agit de préférence d'un radical éthylénique comme par exemple, d'un radical allyle ou 1,1-diméthylallyle, ou d'un radical acétylénique comme, par exemple, le radical éthynyle ou propynyle.

Lorsque R ou $R_1$ représente un radical alkyle cyclique, il s'agit de préférence du radical cyclopropyle, cyclobutyle, cyclopentyle ou cyclohexyle.

Lorsque R représente un radical aralkyle, il s'agit de préférence du radical benzyle.

$alc_1$ et $alc_2$ représentent de préférence un radical méthyle, éthyle, n-propyle, isopropyle, n-butyle, sec-butyle ou isobutyle.

Les produits de formule (I) dans laquelle R représente un radical alkyle et $R_2$ un atome d'hydrogène, notamment la 1-méthyl 4-acétyl 1,2,5,6-tétrahydropyridin-oxime, sont des produits connus pour leurs propriétés parasympathomimétiques (voir à ce sujet le brevet américain n° 3 004 979).

Les produits de l'invention présentent des propriétés pharmacologiques tout à fait inattendues au vu de ce brevet américain, qui sont très intéressantes comme le montrent les résultats des tests biologiques résumés ci-après dans la partie expérimentale.

L'invention a notamment pour objet les composés de formule (I) dans lesquels $R_1$ représente un radical alkyle linéaire renfermant de 1 à 4 atomes de carbone par exemple, le radical méthyle.

Parmi les composés préférés de l'invention, on peut citer les composés de formule (I) dans lesquels R représente un atome d'hydrogène ainsi que ceux dans lesquels R représente un radical hydroxyle et ceux dans lesquels R représente un radical alkyle linéaire ou ramifié, saturé ou insaturé renfermant de 1 à 4 atomes de carbone comme, par exemple, un radical méthyle, éthyle, propyle ou allyle ainsi que leurs sels d'addition avec les acides.

On peut encore citer les composés de formule (I) dans lesquels $R_2$ représente un atome d'hydrogène, ceux dans lesquels $R_2$ représente un radical alkyle renfermant de 1 à 4 atomes de carbone et notamment un radical méthyle.

L'invention a naturellement plus particulièrement pour objet les composés dont la préparation est donnée ci-après dans la partie expérimentale et tout spécialement les produits des exemples 2, 3 et 12.

Les produits de l'invention présentent notamment une activité cholinomimétique par voie orale importante et de longue durée d'action.

Les produits présentent de plus une forte dissociation entre l'activité centrale et l'activité périphérique comme le montrent les résultats des tests exposés ci-après.

L'invention a donc pour objet les produits de l'invention en tant que médicaments, utiles notamment dans le traitement de la maladie d'Alzheimer ou de la démence sénile et également dans le traitement des troubles de la mémoire.

Il est bien connu que les troubles de l'apprentissage et de la mémoire chez les personnes âgées sont surtout reliées à un déficit du système cholinergique central, en particulier dans la démence sénile et la maladie d'Alzheimer.

Il est donc évident que les produits ayant une action cholinergique centrale puissent être employés dans le traitement thérapeutique de ces maladies (Bartus, R.I. Science 217, 408, 1982).

Il a été démontré que l'arécoline injectée par voie intraveineuse a un effet positif sur des patients ayant un déficit de la mémoire (Sitaram N. et al. Science 201, 274, 1978), (Christie J.E. et al. Brit. J. Psychiatry 138, 46, 1981).

Une limitation à l'emploi thérapeutique de l'arécoline est liée au fait que ce produit a une très faible activité par voie orale et une courte durée d'action.

Les produits, objet de l'invention, ont démontré, après administration par voie orale, une activité cholinomimétique centrale très supérieure à celle de l'arécoline et avec une plus longue durée d'action.

La posologie usuelle est variable selon l'affection en cause, le sujet traité et la voie d'administration, elle peut être comprise entre 50 mg et 300 mg/jour, par exemple, entre 15 et 150 mg/jour en une ou plusieurs prises pour le produit de l'exemple 3 administré par voie orale.

La présente invention a également pour objet les compositions pharmaceutiques renfermant comme principe actif au moins un produit de formule (I). Les compositions pharmaceutiques de l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des produits de formule (I) dans laquelle $R_1$ et $R_2$ ont la signification indiquée précédemment et R a la signification indiquée précédemment à l'exception de la valeur hydroxyle, caractérisé en ce que l'on soumet un composé de formule (II) :

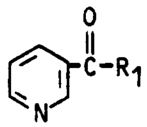

(II)

dans lequel $R_1$ conserve la même signification que précédemment, à l'action d'un composé de formule (III) :

$NH_2OR'_2$ (III)

ou de l'un de ses sels, dans laquelle $R'_2$ représente un atome d'hydrogène ou un radical alkyle linéaire ou ramifié, saturé ou insaturé renfermant jusqu'à 8 atomes de carbone, pour obtenir le composé de formule (IV) :

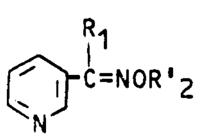

(IV)

que l'on soumet à l'action d'un halogénure d'alkyle de formule (V) :

R′-Hal (V)

dans laquelle Hal représente un atome d'halogène et R′ représente un radical alkyle linéaire, ramifié ou cyclique, saturé ou insaturé renfermant jusqu'à 8 atomes de carbone éventuellement substitué par un radical carboxy libre ou estérifié ou R′ représente un radical aralkyle renfermant jusqu'à 10 atomes de carbone, pour obtenir le composé de formule (VI) :

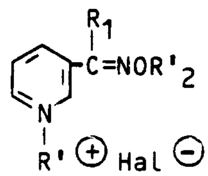

(VI)

que l'on soumet à l'action d'un agent d'hydrogénation, pour obtenir le composé de formule ($I_A$) :

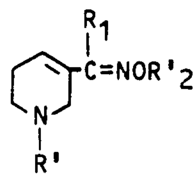

($I_A$)

dans laquelle R′, $R_1$ et $R'_2$ sont définis comme précédemment que, le cas échéant, soit l'on salifie, soit l'on soumet dans le cas ou $R'_2$ représente un atome d'hydrogène, à l'action d'un composé de formule :

$R''_2$-Hal

dans laquelle Hal représente un atome d'halogène et $R''_2$ représente un radical $COalc_1$ ou un radical $-(CH_2)_2N(alc_2)_2$, $alc_1$ et $alc_2$ étant définis comme précédemment, pour obtenir le composé de formule ($I_B$) correspondant :

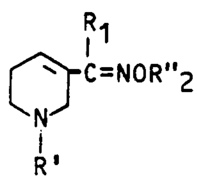

($I_B$)

dans laquelle $R_1$, R′ et $R''_2$ sont définis comme précédement que, le cas échéant, l'on salifie, ou dans le cas où R′ représente un radical aralkyle l'on soumet le produit de formule ($I_A$) ou ($I_B$) à l'action d'un agent de clivage, pour obtenir le composé de formule ($I_C$) :

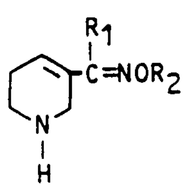

($I_C$)

dans laquelle $R_1$ et $R_2$ sont définis comme précédemment, que le cas échéant, l'on salifie.

Dans un mode de réalisation préférée du procédé de l'invention :

- le composé de formule (III) est utilisé sous forme de chlorhydrate,

- Hal (dans le composé de formule R'-Hal ou $R"_2$-Hal) représente un atome de brome ou d'iode,
- l'agent d'hydrogénation est l'hydroborure de sodium,
- l'agent de clivage est le chlorure d'alpha-chloroéthoxycarbonyle,
- l'halogénoformiate que l'on utilise est un chloroformiate, par exemple d'éthyle, de benzyle.

L'invention a également pour objet une variante du procédé précédent caractérisée en ce que l'on soumet un composé de formule ($I'_A$) :

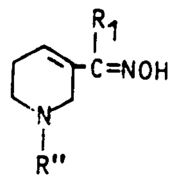

($I'_A$)

dans laquelle R" représente un radical aralkyle renfermant jusqu'à 10 atomes de carbone et $R_1$ conserve sa signification précédente à l'action d'un agent de silylation, pour obtenir le composé de formule (VII) :

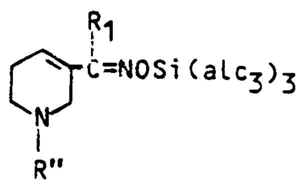

(VII)

dans laquelle $alc_3$ représente un radical alkyle renfermant de 1 à 8 atomes de carbone, que l'on soumet à l'action d'un agent de clivage, pour obtenir le composé de formule ($I_E$) :

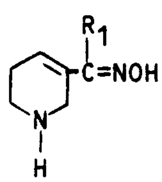

($I_E$)

dans laquelle $R_1$ est défini comme précédemment, que le cas échéant, l'on salifie.

Dans un mode de réalisation préférée :

- R" représente un radical benzyle,
- l'agent de silylation est le chlorure de triméthylsilyle,
- l'agent de clivage est le chlorure d'alpha-chloroéthoxycarbonyle.

L'invention a également pour objet un procédé pour préparer les produits de formule (I) dans laquelle $R_1$ et $R_2$ ont la signification indiquée précédemment et R représente un radical hydroxyle caractérisé en ce que l'on soumet un composé de formule (VIII) :

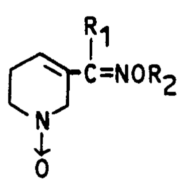

(VIII)

à l'action d'un agent réducteur pour obtenir un composé de formule ($I_F$) :

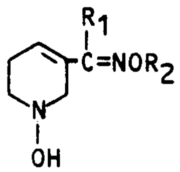

(I_F)

dans laquelle $R_1$ et $R_2$ sont définis comme précédemment que, le cas échéant, l'on salifie.

Dans un mode de réalisation préférée du procédé de l'invention :

- l'agent réducteur est l'hydroborure de sodium.

La salification éventuelle des produits de formule (I) est effectuée selon les méthodes usuelles, en faisant réagir en proportions sensiblement stoéchiometriques, un acide minéral ou organique sur lesdits produits de formule (I).

Les composés de formule (II) sont des produits connus d'une façon générale, qui peuvent être préparés selon le procédé décrit dans le brevet américain n¤ 3 004 979.

Les composés de formule (VIII) sont des produits décrits ou obtenus selon le procédé décrit dans J. Het. Chem., 16, 1459, (1979).

Les exemples suivants illustrent l'invention sans toutefois la limiter.

**Exemple 1** : **3-acétyl 1,2,5,6-tétrahydropyridineoxime et son chlorhydrate.**

**Stade A** : Bromure de 1-benzyl 3-acétyl pyridine oxime.

On dissout 7,4 g de 3-acétylpyridine oxime dans 80 $cm^3$ d'éthanol, ajoute 8 cm3 de bromure de benzyle et chauffe au reflux pendant 6 heures. On élimine le solvant et cristallise dans un mélange éther/méthanol. Onobtient 14,21 g de produit attendu. F = 200-201¤C.

| Analyse : | | | |
|---|---|---|---|
| Calculé :<br>Trouvé : | C% 54,74<br>54,56 | H% 4,92<br>4,98 | N% 9,12<br>9,07 |

**Stade B** : 1-benzyl 3-acétyl 1,2,5,6-tétrahydropyridine oxime.

On refroidit à 0¤C 13,76 g de produit obtenu au stade A en solution dans 100 cm3 de méthanol, ajoute 2,54 g d'hydroborure de sodium, laisse revenir à température ambiante et agite pendant 45 minutes. On concentre sous pression réduite, ajoute de l'eau et extrait au chloroforme. On sèche la phase organique, élimine le solvant, chromatographie le résidu sur silice (éluant : acétate d'éthyle-toluène 8-2) et obtient après cristallisation du résidu dans l'acétate d'éthyle 7,8 g de produit attendu. F = 103-105¤C.

| Analyse : | | | |
|---|---|---|---|
| Calculé :<br>Trouvé : | C% 73,01<br>72,74 | H% 7,88<br>7,81 | N% 12,16<br>12,04 |

**Stade C** : 1-benzyl 3-acétyl 1,2,5,6-tétrahydropyridine triméthylsilyl oxime.

On dissout 3,6 g de produit obtenu au stade B dans 60 cm3 de benzène, 1,86 g de 1,4-diazabicyclo [2.2.2.] octane et ajoute en 5 minutes, sous atmosphère inerte, 2;07 cm3 de triméthylchlorosilane. On chauffe au reflux pendant 3 heures la suspension, refroidit, filtre et concentre à sec. On reprend le résidu dans l'éther éthylique, filtre, élimine le solvant sous pression réduite et obtient 4,5 g de produit attendu. (Eb. : 150¤C sous 0,05 mbar).

| Analyse : | | | |
|---|---|---|---|
| Calculé :<br>Trouvé : | C% 67,50<br>67,33 | H% 8,66<br>8,59 | N% 9,26<br>9,19 |

**Stade D** : 3-acétyl 1,2,5,6-tétrahydropyridine oxime et son chlorhydrate.

On refroidit à 0¤C sous atmosphère inerte 20 g de produitobtenu comme au stade C en solution dans 20 cm3 de chlorure de méthylène et ajoute 21,6 g de chloroformiate d'alpha-chloroéthyle. On chauffe au reflux pendant 2 heures et demie, refroidit, filtre, élimine le solvant, reprend le résidu dans l'éther éthylique, triture et filtre. On évapore le solvant, reprend le résidu dans le méthanol, chauffe au reflux pendant 30 minutes, concentre à sec, reprend le résidu dans le méthanol et l'éther éthylique, filtre, cristallise dans l'éthanol et recueille 2,1 g de produit attendu. F = 237¤C (décomposition).

| Analyse : $C_7H_{12}N_2O$, HCl | | | |
|---|---|---|---|
| Calculé :<br>Trouvé : | C% 47,59<br>47,74 | H% 7,42<br>7,38 | N% 15,86<br>15,78 |

**Exemple 2** : **1-méthyl 3-acétyl 1,2,5,6-tétrahydropyridine O-méthyl oxime et son chlorhydrate.**

**Stade A** : 3-acétyl pyridine O-méthyl oxime.

On ajoute 6,85 g de chlorhydrate de méthoxylamine à 10 g de 3-acétyl pyridine dans 50 cm3 de méthanol. On chauffe au reflux pendant 3 heures, élimine le solvant sous pression réduite, reprend le résidu dans l'eau, alcalinise à l'aide de carbonate de potassium et extrait à l'acétate d'éthyle. On évapore et recueille 11 g de produit attendu. (Eb. : 115-118¤C sous 18 mm Hg).

**Stade B** : Iodure de 1-méthyl 3-acétyl pyridine O-méthyl oxime.

On ajoute 20,5 g d'iodure de méthyle à 11 g du produit obtenu au stade A dans 110 cm3 d'acétate d'éthyle et chauffe 3 heures au reflux. On refroidit, filtre et cristallise le produit dans l'éthanol. On obtient 19,3 g de produit attendu. F = 155-157¤C.

**Stade C** : 1-méthyl 3-acétyl 1,2,5,6-tétrahydropyridine O-méthyl oxime et son chlorhydrate.

On ajoute 1,7 g d'hydroborure de sodium dans une solution de 10 g de produit obtenu au stade B dans 100 cm3 de méthanol, refroidit à +5/+10¤C. On agite 1 heure à température ambiante, élimine le solvant sous pression réduite, reprend le résidu dans l'eau, extrait à l'éther éthylique, évapore à sec, reprend à l'éther éthylique, filtre sur charbon actif et salifie à l'aide d'acide chlorhydrique gazeux. On recristallise le sel dans un mélange isopropanol-éther éthylique et obtient 2,8 g de produit attendu. F = 169-171¤C.

| Analyse : $C_9H_{16}N_2O$, HCl | | | |
|---|---|---|---|
| Calculé :<br>Trouvé : | C% 52,80<br>53,06 | H% 8,37<br>8,44 | N% 13,68<br>13,57 |

**Exemple 3** : **3-acétyl 1,2,5,6-tétrahydropyridine O-méthyl oxïme et son chlorhydrate**.

**Stade A** : Bromure de 1-benzyl 3-acétyl pyridine O-méthyl oxime.

On ajoute 27,8 cm3 de bromure de benzyle à 23,4 g de produit préparé au stade A de l'exemple 2 en solution dans 200 cm3 d'acétate d'éthyle. On chauffe au reflux pendant 8 heures, refroidit, filtre et cristallise le solide dans l'éthanol. On obtient 46 g de produit attendu. F = 191-192¤C.

| Analyse : | | | |
|---|---|---|---|
| Calculé :<br>Trouvé : | C% 56,09<br>56,24 | H% 5,34<br>5,37 | N% 8,72<br>8,67 |

**Stade B** : 1-benzyl 3-acétyl 1,2,5,6-tétrahydropyridine O-méthyl oxime.

On refroidit à 0¤C, 20 g de produit préparé au stade A en solution dans 150 cm3 de méthanol. On ajoute à cette température 3,6 g de hydroborure de sodium, agite 1 heure à température ambiante, élimine le méthanol sous pression réduite, reprend le résidu à l'eau, ajoute du carbonate de sodium jusqu'à saturation, extrait à l'éther éthylique, sèche la phase organique et évapore le solvant. On obtient 10,66 g de produit attendu. (Eb : 128-130¤C sous 0,4 mbar).

| Analyse : | | | |
|---|---|---|---|
| Calculé :<br>Trouvé : | C% 73,74<br>73,56 | H% 8,25<br>8,21 | N% 11,47<br>11,52 |

**Stade C** : 1alpha-chloroéthoxycarbonyl 3-acétyl 1,2,5,6-tétrahydropyridine O-méthyl oxime.

On refroidit à 0¤C 7,2 g de produit obtenu au stade B en solution dans 100 cm3 de dichloroéthane et ajoute en 20 minutes 6,05 g de chloroformiate d'alpha-chloroéthyl dissous dans du dichloroéthane. On chauffe au reflux pendant 1 heure et demie, refroidit, filtre et élimine le solvant sous pression réduite. On reprend le résidu dans l'éther éthylique et filtre de nouveau. On évapore le solvant et obtient 8,44 g de produit que l'on utilise tel quel pour le stade suivant. (Eb. : 210¤C sous 0,06 mbar).

| Analyse : | | | |
|---|---|---|---|
| Calculé :<br>Trouvé : | C% 50,68<br>50,86 | H% 6,57<br>6,46 | N% 10,75<br>10,59 |

**Stade D** : 3-acétyl 1,2,5,6-tétrahydropyridine O-méthyl oxime et son chlorhydrate.

On chauffe au reflux pendant 2 heures le produit obtenu au stade C dans 70 cm3 de méthanol, refroidit, évapore à sec et cristallise le résidu dans l'éthanol. On obtient 3,9 g de produit attendu. F = 199-200¤C. On opère comme à l'exemple 2 et obtient le chlorhydrate attendu.

| Analyse : | | | |
|---|---|---|---|
| Calculé :<br>Trouvé : | C% 50,39<br>50,31 | H% 7,93<br>7,84 | N% 14,69<br>14,55 |

**Exemple 4** : **1-méthyl 3-acétyl 1,2,5,6-tétrahydropyridine O-éthyl oxime et son chlorhydrate.**

**Stade A** : 3-acétyl pyridine O-éthyl oxime.

On dissout 4,8 cm3 de 3-acétyl pyridine dans 50 cm3 de méthanol. On ajoute 4,27 g de chlorhydrate d'orthoéthyl hydroxylamine. On porte au reflux 3 heures. On refroidit et évapore à sec sous pression réduite. On reprend à l'eau, neutralise au bicarbonate de sodium et extrait à l'acétate d'éthyle, sèche et concentre à sec sous pression réduite. On obtient 6,9 g de produit attendu utilisable pour le stade suivant. F = 160-162¤C cristallisé du mélange ether-isopropanol sous forme de chlorhydrate.

**Stade B** : Iodure de 1-méthyl 3-acétyl pyridine O-éthyl oxime.

On agite 8 heures au reflux un mélange de 5,3 g de produit obtenu au stade A et 4,1 cm3 d'iodure de méthyle dans 80 cm3 d'éthanol. On distille à sec. On obtient 9,3 g de produit attendu. F = 95¤C cristallisé d'un mélange éther-isopropanol.

**Stade C** : 1-méthyl 3-acétyl 1,2,5,6-tétrahydropyridine O-éthyl oxime et son chlorhydrate.

A une solution de 9,1 g de produit obtenu au stade B dans 90 cm3 de méthanol, maintenue à 5¤C, on ajoute 1,7 g d'hydrure de bore et de sodium. Après 4 heures à température ambiante, on évapore à sec sous pression réduite. On reprend le résidu à l'eau, extrait à l'acétate d'éthyle et concentre à sec sous pression réduite. Le résidu est chromatographié sur gel de silice (éluant : chloroforme-méthanol 5-1). On dissout dans l'éther les 3,5 g d'huile obtenue et salifie avec de l'acide chlorhydrique gazeux. On évapore à sec et obtient 3,7 g de produit attendu. F = 186-188¤C recristallisé d'un mélange isopropanol-éther.

| Analyse : | | | |
|---|---|---|---|
| Calculé :<br>Trouvé : | C% 54,91<br>54,88 | H% 8,76<br>8,94 | N% 12,81<br>12,76 |

**Exemple 5** : **Chlorhydrate de 3-acétyl 1,2,5,6-tétrahydropyridine O-éthyl oxime et son chlorhydrate.**

**Stade A** : Bromure de 1-N-benzyl 3-acétyl pyridine O-éthyl oxime.

On dissout 6,9 g du produit obtenu au stade A de l'exemple 4 dans 70 cm3 d'éthanol, ajoute 6 cm3 de bromure de benzyle, chauffe au reflux pendant 6 heures, refroidit, évapore sous pression réduite et isole de l'éther 13,9 g de produit attendu.

**Stade B** : 1-N-benzyl 3-acétyl 1,2,5,6-tétrahydropyridine O-éthyl oxime.

a) A une solution de 0,8 g de sodium dans 50 $cm^3$ d'éthanol, on ajoute 3,8 g de produit obtenu au stade B de l'exemple 1 et 1,25 cm3 de bromoéthane. Après 3 heures au reflux, on refroidit et concentre à sec. On isole 2,84 g de produit attendu après élution sur colonne (éluant : acétate d'éthyle-toluène 3-2). (Eb. : 180-190¤C sous 0,05 mbar).
b) Le produit peut aussi être obtenu avec un rendement de 71% par réduction à l'hydrure de bore et de sodium en opérant comme au stade B de l'exemple 1 à partir du produit obtenu au stade A de l'exemple 5.

**Stade C** : 3-acétyl 1,2,5,6-tétrahydropyridine O-éthyl oxime et son chlorhydrate.

On dissout 2,7 g de produit obtenu au stade B dans 50 cm3 de dichloroéthane. On ajoute à 0¤C 1,8 g d'alpha-chloroéthyl chloroformiate. Après 1 heure 30 minutes de reflux, on évapore à sec, reprend à l'éther, filtre l'insoluble et évapore à sec sous pression réduite. On reprend l'huile résiduelle avec 40 cm3 de méthanol. Après 1 heure 30 minutes de reflux, on évapore à sec, reprend le résidu à l'éther, filtre et isole 4,2 g de produit attendu cristallisé d'un mélange éther-méthanol. F = 198-199¤C (décomposition). On opère comme à l'exemple 2 et obtient le chlorhydrate attendu.

| Analyse : | | | |
|---|---|---|---|
| Calculé :<br>Trouvé : | C% 52,81<br>52,59 | H% 8,37<br>8,25 | N% 13,69<br>13,48 |

**Exemple 6** : **3-acétyl 1,2,5,6-tétrahydropyridine O-2-propynyl oxime et son chlorhydrate.**

**Stade A** : 1-N-benzyl 3-acétyl 1,2,5,6-tétrahydropyridine O-2-propynyl oxime.

A une solution de 1,1 g de sodium dans 50 cm3 d'éthanol, on ajoute 5 g de produit obtenu au stade B de l'exemple 1. On ajoute à 0¤C 3,95 g de bromure de propargyle. On chauffe 3 heures à 40¤C, filtre l'insoluble, évapore le solvant et chromatographie sur silice (éluant : acétate d'éthyle). On obtient 4,5 g de produit attendu.

**Stade B** : Chlorhydrate de 3-acétyl 1,2,5,6-tétrahydropyridine O-2-propynyl oxime.

On dissout 7,5 g de produit obtenu au stade A dans 100 cm3 de dichloroéthane. Après 1 heure au reflux, on évapore le solvant, reprend à l'éther, filtre l'insoluble, évapore à sec. On reprend le résidu avec du méthanol, porte au reflux pendant 30 minutes, évapore à sec, reprend le résidu avec une solution de bicarbonate de sodium, extrait à l'acétate d'éthyle et évapore à sec. Le résidu repris à l'éther est salifié avec de l'acide chlorhydrique gazeux. On obtient 1,25 g de produit attendu. F = 178¤C isolé d'un mélange éther-éthanol.

| Analyse : | | | |
|---|---|---|---|
| Calculé :<br>Trouvé : | C% 55,94<br>55,72 | H% 7,04<br>7,01 | N% 13,05<br>12,99 |

**Exemple 7** : **1-méthyl 3-acétyl tétrahydropyridine O-2-propynyl oxime et son chlorhydrate.**

**Stade A** : 3-acétyl pyridine O-2-propynyl oxime.

On dissout 5 g de 3-acétyl pyridine dans 20 cm3 d'eau, ajoute 3,45 g de bicarbonate de sodium et 4,45 g de chlorhydrate d'ortho-2-propynyl hydroxylamine dans 30 cm3 d'eau. On laisse 16 heures puis chauffe 3 heures à 40¤C. On concentre, extrait à l'acétate d'éthyle, évapore à sec et purifie sur gel de silice (éluant : acétate d'éthyle). On obtient 5,7 g de produit attendu. F = 156-157¤C cristallisé de l'isopropanol.

**Stade B** : Iodure de 1-N-méthyl 3-acétyl pyridine O-2-propynyl oxime.

On dissout 5,6 g de produit obtenu au stade A dans 60 cm3 de méthanol et ajoute 8,6 g d'iodure de méthyle. Après 3 heures de reflux, on évapore à sec, reprend avec un mélange d'acétone et d'éther et filtre. On obtient 9,7 g. F = 150-151¤C cristallisé de l'isopropanol.

**Stade C** : 1-méthyl 3-acétyl tétrahydropyridine O-2-propynyl oxime et son chlorhydrate.

On dissout 9,7 g de produit obtenu au stade B dans 100 cm3 de méthanol, ajoute 2,35 g d'hydrure de bore et de sodium à 0¤C. Après 1 heure à 0¤C, on évapore, reprend le résidu à l'eau, extrait à l'acétate d'éthyle, sèche et évapore à sec. On salifie le résidu dans l'éther avec de l'acide chlorhydrique gazeux et obtient 2,7 g de produit attendu. F = 195-196¤C recristallisé d'un mélange isopropanol-éther.

| Analyse : $C_{11}H_{16}N_2O$, HCl | | | |
|---|---|---|---|
| Calculé :<br>Trouvé : | C% 57,76<br>57,59 | H% 7,49<br>7,38 | N% 12,25<br>12,11 |

**Exemple 8** : **Chlorhydrate de 3-propionyl 1,2,5,6-tétrahydropyridine oxime.**

**Stade A** : Bromure de 1-N-benzyl 3-propionyl pyridine oxime.

On maintient au reflux pendant 7 heures 30 minutes un mélange de 17,8 g de 3-propionyl pyridine oxime [US Pat. 3 004 979 (1961)] et de 18 cm3 de bromure de benzyle dans 250 cm3 d'acétate d'éthyle. On refroidit, essore et obtient 36,5 g de produit attendu. F = 178-180¤C cristallisé d'un mélange éthanol-éther.

**Stade B** : 1-N-benzyl 3-propionyl 1,2,5,6-tétrahydropyridine oxime.

On dissout 36,2 g de produit obtenu au stade A dans 250 cm3 de méthanol, maintient la température à 10° C en ajoutant par portions 6,4 g d'hydrure de bore et de sodium. On agite 3 heures, évapore sous pression réduite, reprend à l'eau, extrait à l'acétate d'éthyle, séche et évapore le solvant. On purifie par chromatographie sur colonne (éluant : acétate d'éthyle) et obtient 21 g de produit attendu. F = 111-112¤C cristallisé de l'acétate d'éthyle.

**Stade C** : 1-N-benzyl 3-propionyl 1,2,5,6-tétrahydropyridine O-triméthyl silyl oxime.

On mélange 6 g de produit obtenu au stade B, 70 cm3 de benzène et 2,95 g de 1,4-diazabicyclo [2.2.2.] octane. On ajoute 3,25 cm3 de chlorure de triméthylsilyle, porte au reflux pendant 3 heures, refroidit, filtre l'insoluble, évapore le solvant, reprend le résidu à l'éther, filtre l'insoluble et évapore à sec. On obtient 7,5 g de produit attendu. (Eb. : 250¤C sous 0,06 mbar).

**Stade D** : 3-propionyl 1,2,5,6-tétrahydropyridine oxime et son chlorhydrate.

On dissout 16 g de produit obtenu au stade C dans 150 cm3 de dichloroéthane. On refroidit à 0¤C et ajoute 16,2 g d'alpha-chloroéthyl chloroformiate. On maintient 3 heures au reflux puis évapore le solvant, reprend à l'éther, filtre et concentre à sec. On reprend le résidu avec 100 cm3 de méthanol, porte au reflux pendant 6 heures. On évapore le méthanol puis purifie par passage sur alumine (éluant : chloroforme-méthanol 7-3 puis méthanol seul). On évapore à sec, reprend par de l'éthanol, filtre sur charbon et précipite par ajout d'hexane. On filtre et obtient, après purification dans un mélange d'éthanol-hexane, 1,3 g de produit attendu. F = 205-206¤C. On opère comme à l'exemple 2 et obtient le chlorhydrate attendu.

| Analyse : $C_8H_{14}N_2O$, HCl | | | |
|---|---|---|---|
| Calculé :<br>Trouvé : | C% 50,39<br>50,08 | H% 7,93<br>7,87 | N% 14,69<br>14,48 |

**Exemple 9** : **3-propionyl 1,2,5,6-tétrahydropyridine O-méthyl oxime et son chlorhydrate.**

**Stade A** : 1-N-benzyl 3-propionyl 1,2,5,6-tétrahydropyridine O-méthyl oxime.

A une solution de 1,2 g de sodium dans 80 cm3 d'éthanol, on ajoute 6,11 g de produit obtenu au stade B de l'exemple 8. On ajoute ensuite 1,58 cm3 d'iodure de méthyle, porte au reflux pendant 6 heures, refroidit, concentre à sec sous pression réduite et reprend à l'eau. On extrait à l'acétate d'éthyle, sèche, évapore à sec, chromatographie le résidu (éluant : acétate d'éthyle-toluène 6-4) et obtient 2,5 g de produit attendu. (Eb : 170¤C à 0,05 mbar).

**Stade B** : 3-propionyl 1,2,5,6-tétrahydropyridine O-méthyl oxime et son chlorhydrate.

On dissout 3,5 g de produit du stade A dans 50 cm3 de dichloroéthane. On ajoute à 0¤C 2,04 g d'alpha-chloroéthyl chloroformiate. Après 3 heures au reflux, on évapore le solvant, reprend le résidu à l'éther, filtre et évapore à sec. On reprend avec 30 cm3 de méthanol et porte au reflux pendant 30 minutes. On évapore à sec, cristallise d'un mélange éthanol-éther et obtient 1,17 g de produit attendu. F = 210-212¤C. On opère comme à l'exemple 2 et obtient le chlorhydrate attendu.

| Analyse : $C_9H_{16}N_2O$, HCl | | | |
|---|---|---|---|
| Calculé :<br>Trouvé : | C% 52,53<br>52,80 | H% 8,26<br>8,37 | N% 13,56<br>13,68 |

**Exemple 10** : **1-N-carboxyéthyl 3-acétyl 1,2,5,6-tétrahydropyridine O-méthyl oxime.**

On dissout 1,8 g de produit obtenu à l'exemple 3 dans 30 cm3 de benzène à 5¤C puis ajoute 1,63 cm3 de triéthylamine et 1,12 cm3 de chloroformiate d'éthyle. On agite 30 minutes à température ambiante, lave à l'eau, évapore à sec et rectifie. On obtient 2,56 g de produit attendu. (Eb : 155¤C sous 0,07 mbar).

| Analyse : | | | |
|---|---|---|---|
| Calculé :<br>Trouvé : | C% 58,39<br>58,41 | H% 8,02<br>7,88 | N% 12,38<br>12,29 |

**Exemple 11** : **1-N-carboxy benzyl 3-acétyl 1,2,5,6-tétrahydropyridine O-méthyl.**

On dissout 2,5 g de produit obtenu au stade B de l'exemple 3 dans 40 cm3 de benzène anhydre, ajoute 2,7 cm3 de chloroformiate de benzyle, porte au reflux pendant 1 heure, refroidit, lave avec 10 cm3 d'acide chlorhydrique à 5%, sèche puis évapore le solvant. On élimine sous le chlorure de benzyle formé et obtient 2,3 g de produit attendu cristallisé du cyclohexane. F = 90¤C.

| Analyse : | | | |
|---|---|---|---|
| Calculé :<br>Trouvé : | C% 65,68<br>65,81 | H% 6,61<br>6,58 | N% 10,21<br>10,17 |

**Exemple 12** : **1-N-hydroxy 3-acétyl 1,2,5,6-tétrahydropyridine O-méthyl oxime.**

On dissout 3,5 g de 3-acétyl pyridine O-méthyl oxime N-oxyde [J. heterocyclic Chem. 16, 1459, (1979)] dans 50 cm3 de méthanol. On refroidit à -10¤C et ajoute 2,4 g d'hydrure de bore et de sodium. On agite 2 heures à température ambiante. On évapore à sec, reprend à l'eau, extrait à l'acétate d'éthyle, sèche, évapore à sec. On purifie par chromatographie sur gel de silice (éluant : acétate d'éthyle). On obtient 2,85 g de produit attendu. F = 94-96¤C cristallisé de l'hexane.

| Analyse : | | | |
|---|---|---|---|
| Calculé :<br>Trouvé : | C% 56,45<br>56,28 | H% 8,29<br>8,32 | N% 16,46<br>16,31 |

**Exemple 13** : **1-méthyl 3-cyclopropylcarbonyl 1,2,5,6-tétrahydropyridine O-méthyl oxime et son benzène sulfonate.**

**Stade A** : 3-pyridyl cyclopropylcétone O-méthyl oxime.

A une solution de 3,5 g de 3-pyridyl cyclopropylcétone dans 50 cm3 de méthanol, on ajoute 2,03 g de chlorhydrate de méthoxylamine. On chauffe au reflux pendant 4 heures, évapore le solvant et reprend avec une solution aqueuse de bicarbonate de sodium. On extrait au chlorure de méthylène, sèche puis évapore. On obtient 4,1 g de produit attendu.

**Stade B** : Iodure de 1-méthyl 3-cyclopropylcarbonyl pyridine O-méthyl oxime.

A une solution de 5,3 g d'iodure de méthyle dans 50 cm3 de méthanol, on ajoute 4 g de produit obtenu au stade A et porte au reflux pendant 3 heures. On évapore à séc, empâte dans un mélange éthanol-éther, filtre et obtient 6,6 g de produit attendu cristallisé de l'isopropanol. F = 133¤C (décomposition).

| Analyse : $C_{11}H_{15}IN_2O$ | | | |
|---|---|---|---|
| Calculé : | C% 41,52 | H% 4,75 | N% 8,80 |
| Trouvé : | 41,17 | 4,66 | 8,76 |

**Stade C** : 1-méthyl 3-cyclopropylcarbonyl 1,2,5,6-tétrahydropyridine O-méthyl oxime et son benzène sulfonate.

A une solution de 6,4 g de produit obtenu au stade B dans 70 cm3 de méthanol, on introduit à 0¤C 1,52 g d'hydrure de bore et de sodium. On laisse réagir pendant 1 heure 30 minutes à température ambiante. On évapore, reprend avec de l'eau, extrait avec de l'acétate d'éthyle, sèche et évapore à sec. On purifie par élution sur alumine (éluant : toluène-acétate d'éthyle 6-4). On distille à 160¤C sous 0,2 mm de mercure. 2,45 g de la base obtenue sont repris dans 100 cm3 de benzène avec 1,994 g d'acide benzène sulfonique. On évapore à sec, empâte dans un peu d'acétate d'éthyle, filtre et obtient 1,7 g de produit attendu. F = 76¤C (décomposition).

| Analyse : $C_{11}H_{18}N_2O$, $C_6H_5SO_3H$ | | | |
|---|---|---|---|
| Calculé : | C% 57,93 | H% 6,86 | N% 7,95 |
| Trouvé : | 58,04 | 6,94 | 7,87 |

**Exemples de compositions pharmaceutiques.**

a) On a préparé des comprimés répondant à la formule suivante :

| | |
|---|---|
| - Produit de l'exemple 3 | 200 mg |
| - Excipient q.s. pour un comprimé terminé à | 300 mg |

(Détail de l'excipient : lactose, amidon de blé, amidon traité, amidon de riz, stéarate de magnésium, talc).

b) On a préparé des gélules répondant à la formule suivante :

| | |
|---|---|
| - Produit de l'exemple 1 | 100 mg |
| - Excipient q.s. pour une gélule terminée à | 300 mg |

(Détail de l'excipient : talc, stéarate de magnésium, aérosil).

c) On a préparé des comprimés répondant à la formule suivante :

| | |
|---|---|
| - Produit de l'exemple 2 | 50 mg |
| - Excipient q.s. pour un comprimé terminé à | 300 mg |

(Détail de l'excipient : lactose, amidon de blé, amidon traité, amidon de riz, stéarate de magnésium, talc).

d) On a préparé des comprimés répondant à la formule suivante :

| | |
|---|---|
| - Produit de l'exemple 12 | 50 mg |
| - Excipient q.s. pour un comprimé terminé à | 300 mg |

(Détail de l'excipient : lactose, amidon de blé, amidon traité, amidon de riz, stéarate de magnésium, talc).

**Activité biologique.**

Les produits ont été utilisés sous forme de chlorhydrate.

**Toxicité aiguë.**

L'essai est réalisé sur des souris mâles ($CD_1$ Charles Rivers) de 22 à 24 g, à jeun depuis 16 heures. On administre les produits par voie orale à la dose 1000, 500, 250, 125, 62 et 31 mg:kg. On note la mortalité pendant les 7 jours suivant le traitement.

| Produit de l'exemple | $DL_{50}$ en mg/kg |
|---|---|
| 1 | 350 |
| 2 | 350 |
| 3 | 125 |
| 12 | 175 |
| Arècoline HBr | 600 |

**Test de l'iléon de cobaye.**

On prélève des fragments d'iléon de cobayes tués par décapitation. L'iléon isolé est placé dans 10 cm3 de solution de Tyrode à 37¤C et aéré par un mélange d'oxygène (95%) et de gaz carbonique (5%). Les contractions dues aux produits sont enregistrées à l'aide d'un capteur relié à un polygraphe. Les produits à tester sont ajoutés aux concentrations comprises entre $1.10^{-3}$M et $1.10^{-8}$ M/l.

Les produits présentant un effet contracturant sont testés vis à vis de l'atropine et de l'hexaméthonium pour établir si l'activité est de type "muscarinique" ou "nicotinique".

L'activité agoniste est exprimée en $pD_2$ (logarithme négatif de la dose qui produit 50% de l'effet maximum).

| Exemple | $pD_2$ |
|---|---|
| 1 | 5,25 |
| 2 | 4,85 |
| 3 | 7,50 |
| 12 | 5,39 |
| Arécoline | 6,48 |

**Activité diarrhéique.**

Le test est réalisé sur des souris mâles ($CD_1$ Charles Rivers) pesant 25 à 30 g, à jeun depuis 6 heures. Le produit dissous à 5% dans du méthocel est administré par voie orale, au moyen d'une sonde oesophagienne.

Des animaux témoins ne reçoivent que l'excipient.

Après traitement, les animaux sont mis séparément dans des cages dont le fond est recouvert de papier buvard et sont mis en observation pendant 30, 60, 120 et 180 minutes.

Les feuilles de papier absorbant sont changées après chaque observation.

La consistance des fèces est évaluée selon la méthode de Randall et Baruth (Arch. Int. Pharmacodyn. 220, 94, 1976) en suivant l'échelle des valeurs suivantes :

0 : consistance ferme,
1 : fèces légèrement molles avec ou sans auréole humide,
2 : fèces légèrement molles avec présence d'un cercle humide bien défini,
3 : fèces molles avec présence d'un grand cercle humide,
4 : fèces sans consistance avec présence d'un très grand cercle humide.

Pour chaque produit, on a noté la dose qui provoque une diarrhée chez 50% des animaux selon la méthode de Miller et Tainter (Proc. Soc. Exp. Biol. Med. 57, 261, 1944).

| Exemple | $DE_{50}$ en mg/kg |
|---|---|
| 1 | 5 |
| 2 | 100 |
| 3 | 0,85 |
| 12 | 1 |
| Arécoline | 35 |

**Activité hypothermique.**

Le test est réalisé sur des souris mâles ($CD_1$ Charles Rivers) pesant 25 à 30 g, à jeun depuis 6 heures.

La température du corps est notée au moyen d'un thermocouple placé dans le rectum à environ 1,5 cm et relié à un enregistreur de température électrique.

Les produits sont administrés par voie orale ou sous-cutanée et les températures sont notées à l'instant 0 et 30 minutes, 1 heure, 2 heures et 2 heures et demie après traitement.

On évalue le degré d'hypothermie comme la différence entre les animaux traités et les témoins et on détermine la dose nécessaire pour réduire de 1¤C la température du corps.

| Exemple | Dose efficace (-1¤C) en mg/kg | |
|---|---|---|
| | V.O | V.SC |
| 1 | 9 | 11 |
| 2 | 12 | 25 |
| 3 | 0,87 | 0,91 |
| 12 | 0,77 | 0,72 |
| Arècoline | 194 | 3 |

La durée d'action des produits a été évaluée en employant la dose qui réduisait la température corporelle de 1¤ - 1,5¤C.

VARIATION DE LA TEMPERATURE CORPORELLE

| Produit de l'exemple | Dose mg/kg | voie administ. | temps en minute après le traitement 0 | 30 | 60 | 120 | 180 |
|---|---|---|---|---|---|---|---|
| 1 | 10 | p.o | 0 | -0,7 | -1,0 | -0,2 | +0,1 |
| | 10 | s.c | 0 | -0,6 | -0,8 | -0,2 | 0 |
| 2 | 20 | p.o | +0,1 | -1,3 | -1,3 | -0,9 | 0 |
| | 40 | s.c | +0,1 | -1,0 | -1,4 | -1,1 | -0,8 |
| 3 | 1 | p.o | +0,1 | -1,1 | -1,0 | -0,1 | -0,1 |
| | 1 | s.c | +0,1 | -1,0 | -0,8 | +0,1 | +0,1 |
| 12 | 1 | p.o | 0 | -1,3 | -1,1 | 0 | 0 |
| | 1 | s.c | -0,1 | -1,4 | -1,3 | +0,1 | 0 |
| Arécoline | 200 | p.o | +0,1 | -1,1 | -1,0 | -0,2 | -0,1 |
| HBr | 3,5 | s.c | -0,1 | -1,5 | -0,1 | +0,2 | +0,2 |

## Revendications

### Revendications pour les Etats contractants suivants : AT, BE, CH, DE, FR, GB, IT, LI, LU, NL, SE

**1.** Les composés de formule (I) :

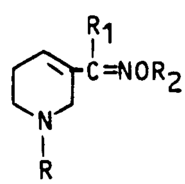

(I)

dans laquelle R représente un atome d'hydrogène, un radical hydroxyle, un radical alkyle linéaire, ramifié ou cyclique, saturé ou insaturé renfermant jusqu'à 8 atomes de carbone, ou R représente un radical aralkyle renfermant jusqu'à 10 atomes de carbone $R_1$ représente un radical alkyle linéaire, ramifié ou cyclique, saturé ou insaturé renfermant jusqu'à 8 atomes de carbone et $R_2$ représente un atome d'hydrogène ou un radical alkyle linéaire ou ramifié, saturé ou insaturé renfermant jusqu'à 8 atomes de carbone, un radical $COalc_1$ ou un radical $(CH_2)_2N(alc_2)_2$, $alc_1$ et $alc_2$ représentant un radical alkyle renfermant jusqu'à 8 atomes de carbone ainsi que leurs sels d'addition avec les acides à la condition que si R représente un radical alkyle, $R_2$ ne représente pas un atome d'hydrogène.

**2.** Les composés de formule (I) tels que définis à la revendication 1, dans lesquels $R_1$ représente un

radical alkyle linéaire renfermant de 1 à 4 atomes de carbone ainsi que leurs sels d'addition avec les acides.

**3.** Les composés de formule (I) tels que définis à la revendication 2, dans lesquels $R_1$ représente un radical méthyle ainsi que leurs sels d'addition avec les acides.

**4.** Les composés de formule (I) tels que définis à l'une quelconque des revendications 1 à 3 dans lesquels R représente un atome d'hydrogène ou un radical hydroxyle ainsi que leurs sels d'addition avec les acides.

**5.** Les composés de formule (I) tels que définis à l'une quelconque des revendications 1 à 3 dans lesquels R représente un radical alkyle linéaire ou ramifié, saturé ou insaturé renfermant de 1 à 4 atomes de carbone ainsi que leurs sels d'addition avec les acides.

**6.** Les composés de formule (I) tels que définis à la revendication 5, dans lesquels R représente un radical méthyle, éthyle, propyle ou allyle ainsi que leurs sels d'addition avec les acides.

**7.** Les composés de formule (I) tels que définis à l'une quelconque des revendications 1 à 6, dans lesquels $R_2$ représente un atome d'hydrogène ainsi que leurs sels d'addition avec les acides.

**8.** Les composés de formule (I) tels que définis à l'une quelconque des revendications 1 à 6, dans lesqels $R_2$ représente un radical alkyle renfermant de 1 à 4 atomes de carbone et notamment un radical méthyle.

**9.** Les composés de formule (I) tels que définis à la revendication 1 dont les noms suivent :
- le 1-méthyl 3-acétyl 1,2,5,6,-tétrahydropyridine O-méthyl oxime et son chlorhydrate,
- le 3-acétyl 1,2,5,6-tétrahydropyridine O-méthyl oxime et son chlorhydrate,
- le 1-N-hydroxyl 3-acétyl 1,2,5,6-tétrahydropyridine O-méthyl oxime.

**10.** Procédé de préparation des composés de formule (I) dans laquelle $R_1$ et $R_2$ ont la signification indiquée précédemment et R a la signification indiquée précédemment à l'exception de la valeur hydroxyle, caractérisé en ce que l'on soumet un composé de formule (II) :

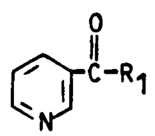

(II)

dans lequel $R_1$ conserve la même signification que précédemment, à l'action d'un composé de formule (III) :

$NH_2OR'_2$ (III)

ou de l'un de ses sels, dans laquelle $R'_2$ représente un atome d'hydrogène ou un radical alkyle linéaire ou ramifié, saturé ou insaturé renfermant jusqu'à 8 atomes de carbone, pour obtenir le composé de formule (IV) :

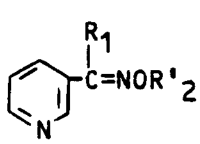

(IV)

que l'on soumet à l'action d'un halogénure d'alkyle de formule (V) :

R'-Hal (V)

dans laquelle Hal représente un atome d'halogène et R' représente un radical alkyle linéaire, ramifié ou cyclique, saturé ou insaturé renfermant jusqu'à 8 atomes de carbone éventuellement substitué par un radical carboxy libre ou estérifié ou R' représente un radical aralkyle renfermant jusqu'à 10 atomes de carbone, pour obtenir le composé de formule (VI) :

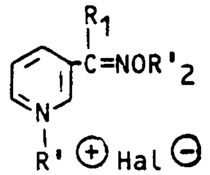

(VI)

que l'on soumet à l'action d'un agent d'hydrogénation, pour obtenir le composé de formule ($I_A$) :

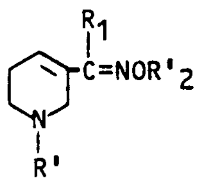

($I_A$)

dans laquelle R', $R_1$ et $R'_2$ sont définis comme précédemment que, le cas échéant, soit l'on salifie, soit l'on soumet dans le cas ou $R'_2$ représente un atome d'hydrogène, à l'action d'un composé de formule :

$R''_2$-Hal

dans laquelle Hal représente un atome d'halogène et $R''_2$ représente un radical $COalc_1$ ou un radical $-(CH_2)_2N(alc_2)_2$, $alc_1$ et $alc_2$ étant définis comme précédemment, pour obtenir le composé de formule ($I_B$) correspondant :

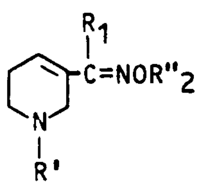

($I_B$)

dans laquelle $R_1$, R' et $R''_2$ sont définis comme précédement que, le cas échéant, l'on salifie, ou dans le cas où R' représente un radical aralkyle l'on soumet le composé de formule ($I_A$) ou ($I_B$) à l'action d'un agent de clivage, pour obtenir le composé de formule ($I_C$) :

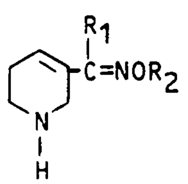

($I_C$)

dans laquelle $R_1$ et $R_2$ sont définis comme précédemment, que le cas échéant, l'on salifie.

**11.** Variante du procédé selon la revendication 10, caractérisée en ce que l'on soumet un composé de formule ($I'_A$) :

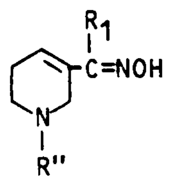

(I'$_A$)

dans laquelle R" représente un radical aralkyle renfermant jusqu'à 10 atomes de carbone et $R_1$ conserve sa signification précédente à l'action d'un agent de silylation, pour obtenir le composé de formule (VII) :

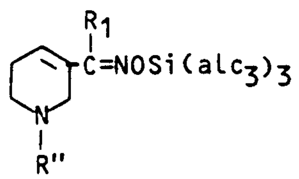

(VII)

dans laquelle $alc_3$ représente un radical alkyle renfermant de 1 à 8 atomes de carbone, que l'on soumet à l'action d'un agent de clivage, pour obtenir le composé de formule ($I_E$) :

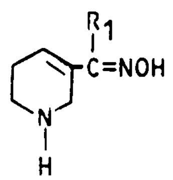

($I_E$)

dans laquelle $R_1$ est défini comme précédemment, que le cas échéant, l'on salifie.

**12.** Procédé de préparation des composés de formule (I) dans laquelle $R_1$ et $R_2$ ont la signification indiquée précédemment et R représenté un radical hydroxyle caractérisé en ce que l'on soumet un composé de formule (VIII) :

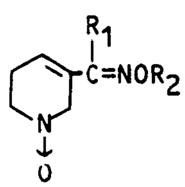

(VIII)

à l'action d'un agent réducteur pour obtenir un composé de formule ($I_F$) :

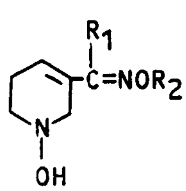

($I_F$)

dans laquelle $R_1$ et $R_2$ sont définis comme précédemment que, le cas échéant, l'on salifie.

13. A titre de médicaments, les composés de formule (I) tels que définis à l'une quelconque des revendications 1 à 9, ainsi que leurs sels d'addition avec les acides pharmaceutiquement acceptables.

14. Les compositions pharmaceutiques renfermant comme principe actif au moins un médicament défini à la revendication 13.

**Revendications pour l'Etat contractant suivant : ES**

1. Procédé pour préparer les composés de formule (I) :

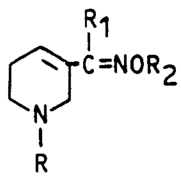

(I)

dans laquelle R représente un atome d'hydrogène, un radical hydroxyle, un radical alkyle linéaire, ramifié ou cyclique, saturé ou insaturé renfermant jusqu'à 8 atomes de carbone, ou R représente un radical aralkyle renfermant jusqu'à 10 atomes de carbone $R_1$ représente un radical alkyle linéaire, ramifié ou cyclique, saturé ou insaturé renfermant jusqu'à 8 atomes de carbone et $R_2$ représente un atome d'hydrogène ou un radical alkyle linéaire ou ramifié, saturé ou insaturé renfermant jusqu'à 8 atomes de carbone, un radical $COalc_1$ ou un radical $(CH_2)_2N(alc_2)_2$, $alc_1$ et $alc_2$ représentant un radical alkyle renfermant jusqu'à 8 atomes de carbone ainsi que leurs sels d'addition avec les acides à la condition que si R représente un radical alkyle, $R_2$ ne représente pas un atome d'hydrogène, caractérisé en ce que :

a) pour préparer les composés de formule (I) dans laquelle $R_1$ et $R_2$ ont la signification indiquée précédemment et R a la signification indiquée précédemment à l'exception de la valeur hydroxyle, l'on soumet un composé de formule (II) :

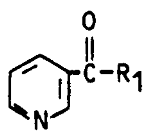

(II)

dans lequel $R_1$ conserve la même signification que précédemment, à l'action d'un composé de formule (III) :

$NH_2OR'_2$ (III)

ou de l'un de ses sels, dans laquelle $R'_2$ représente un atome d'hydrogène ou un radical alkyle linéaire ou ramifié, saturé ou insaturé renfermant jusqu'à 8 atomes de carbone, pour obtenir le composé de formule (IV) :

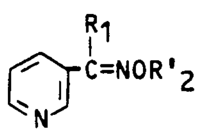

(IV)

que l'on soumet à l'action d'un halogénure d'alkyle de formule (V) :

R'-Hal (V)

dans laquelle Hal représente un atome d'halogène et R' représente un radical alkyle linéaire, ramifié ou cyclique, saturé ou insaturé renfermart jusqu'à 8 atomes de carbone éventuellement substitué par un radical carboxy libre ou estérifié ou R' représente un radical aralkyle renfermant jusqu'à 10 atomes de carbone, pour obtenir le composé de formule (VI) :

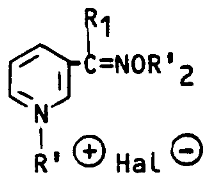

(VI)

que l'on soumet à l'action d'un agent d'hydrogénation, pour obtenir le composé de formule ($I_A$) :

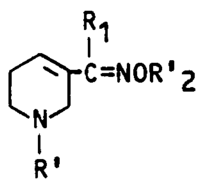

($I_A$)

dans laquelle R', $R_1$ et $R'_2$ sont définis comme précédemment que, le cas échéant, soit l'on salifie, soit l'on soumet dans le cas ou $R'_2$ représente un atome d'hydrogène, à l'action d'un composé de formule :

$R''_2$-Hal

dans laquelle Hal représente un atome d'halogène et $R''_2$ représente un radical $COalc_1$ ou un radical $-(CH_2)_2N(alc_2)_2$, $alc_1$ et $alc_2$ étant définis comme précédemment, pour obtenir le composé de formule ($I_B$) correspondant :

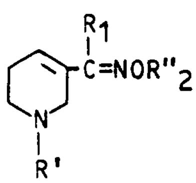

($I_B$)

dans laquelle $R_1$, R' et $R''_2$ sont définis comme précédement que, le cas échéant, l'on salifie, ou dans le cas où R' représente un radical aralkyle l'on soumet le composé de formule ($I_A$) ou ($I_B$) à l'action d'un agent de clivage, pour obtenir le composé de formule ($I_C$) :

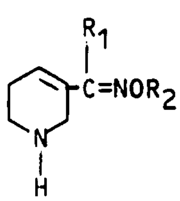

($I_C$)

dans laquelle $R_1$ et $R_2$ sont définis comme précédemment, que le cas échéant, l'on salifie.
b) pour préparer les composés de formule (I) dans laquelle $R_1$ et $R_2$ ont la signification indiquée

précédemment et R représente un radical hydroxyle l'on soumet un composé de formule (VIII) :

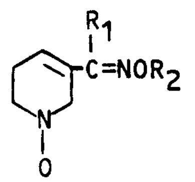

(VIII)

à l'action d'un agent réducteur pour obtenir un composé de formule ($I_F$) :

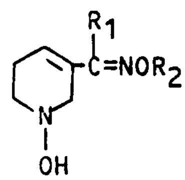

($I_F$)

dans laquelle $R_1$ et $R_2$ sont définis comme précédemment que, le cas échéant, l'on salifie.

**2.** Procédé selon la revendication 1, pour préparer les composés de formule (I) dans laquelle $R_1$ et $R_2$ ont la signification indiquée précédemment et R a la signification indiquée précédemment à l'exception de la valeur hydroxyle, caractérisé en ce que l'on soumet un composé de formule (II) :

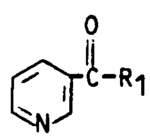

(II)

dans lequel $R_1$ conserve la même signification que précédemment, à l'action d'un composé de formule (III) :

$NH_2OR'_2$ (III)

ou de l'un de ses sels, dans laquelle $R'_2$ représente un atome d'hydrogène ou un radical alkyle linéaire ou ramifié, saturé ou insaturé renfermant jusqu'à 8 atomes de carbone, pour obtenir le composé de formule (IV) :

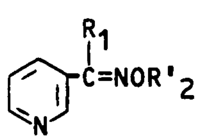

(IV)

que l'on soumet à l'action d'un halogénure d'alkyle de formule (V) :

R'-Hal (V)

dans laquelle Hal représente un atome d'halogène et R' représente un radical alkyle linéaire, ramifié ou cyclique, saturé ou insaturé renfermant jusqu'à 8 atomes de carbone éventuellement substitué par un radical carboxy libre ou estérifié ou R' représente un radical aralkyle renfermant jusqu'à 10 atomes de carbone, pour obtenir le composé de formule (VI) :

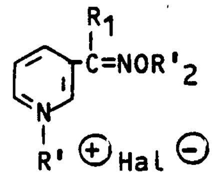

(VI)

que l'on soumet à l'action d'un agent d'hydrogénation, pour obtenir le composé de formule ($I_A$) :

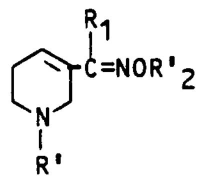

($I_A$)

dans laquelle R', $R_1$ et $R'_2$ sont définis comme précédemment que, le cas échéant, soit l'on salifie, soit l'on soumet dans le cas ou $R'_2$ représente un atome d'hydrogène, à l'action d'un composé de formule :

$R''_2$-Hal

dans laquelle Hal représente un atome d'halogène et $R''_2$ représente un radical $COalc_1$ ou un radical $-(CH_2)_2N(alc_2)_2$, $alc_1$ et $alc_2$ étant définis comme précédemment, pour obtenir le composé de formule ($I_B$) correspondant :

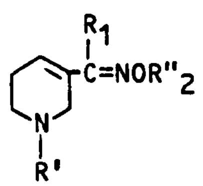

($I_B$)

dans laquelle $R_1$, R' et $R''_2$ sont définis comme précédement que, le cas échéant, soit l'on salifie, soit dans le cas où R' représente un radical aralkyle l'on soumet à l'action d'un agent de clivage, pour obtenir le composé de formule ($I_C$) :

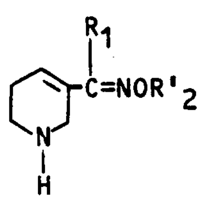

($I_C$)

dans laquelle $R_1$ et $R'_2$ sont définis comme précédemment, que le cas échéant, l'on salifie.

**3.** Procédé selon la revendication 2, pour préparer les composés de formule (I) telle que définie à la revendication 1, répondant à la formule ($I_A$) :

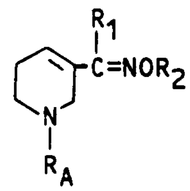

$(I_A)$

dans laquelle $R_1$ et $R_2$ sont définis comme à la revendication 1 et $R_A$ représente un atome d'hydrogène, un radical alkyle linéaire, ramifié ou cyclique, saturé ou insaturé renfermant jusqu'à 8 atomes de carbone, éventuellement substitué par un radical carboxy libre ou estérifié ou R représente un radical aralkyle renfermant jusqu'à 10 atomes de carbone ainsi que leurs sels d'addition avec les acides à la condition que si $R_A$ représente un radical alkyle, $R_2$ ne représente pas un atome d'hydrogène, caractérisé en ce que l'on soumet un composé de formule (II) :

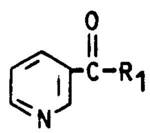

(II)

dans lequel $R_1$ conserve la même signification que précédemment, à l'action d'un composé de formule (III) :

$NH_2OR'_2$ (III)

ou de l'un de ses sels, dans laquelle $R'_2$ représente un atome d'hydrogène ou un radical alkyle linéaire ou ramifié, saturé ou insaturé renfermant jusqu'à 8 atomes de carbone, pour obtenir le composé de formule (IV) :

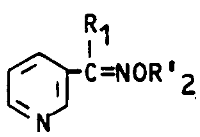

(IV)

que l'on soumet à l'action d'un halogénure d'alkyle de formule (V) :

R'-Hal (V)

dans laquelle Hal représente un atome d'halogène et R' représente un radical alkyle linéaire, ramifié ou cyclique, saturé ou insaturé renfermant jusqu'à 8 atomes de carbone éventuellement substitué par un radical carboxy libre ou estérifié ou R' représente un radical aralkyle renfermant jusqu'à 10 atomes de carbone, pour obtenir le composé de formule (VI) :

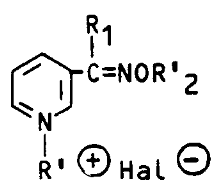

(VI)

que l'on soumet à l'action d'un agent d'hydrogénation, pour obtenir le composé de formule $(I_A)$ :

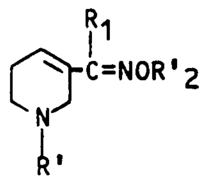

(I_A)

dans laquelle R', $R_1$ et $R'_2$ sont définis comme précédemment que, le cas échéant, soit l'on salifie, soit l'on soumet dans le cas ou $R'_2$ représente un atome d'hydrogène, à l'action d'un composé de formule :

$R''_2$-Hal

dans laquelle Hal représente un atome d'halogène et $R''_2$ représente un radical $COalc_1$ ou un radical $-(CH_2)_2N(alc_2)_2$, $alc_1$ et $alc_2$ étant définis comme précédemment, pour obtenir le composé de formule ($I_B$) correspondant :

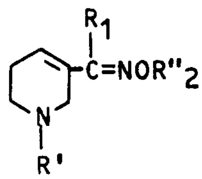

(I_B)

dans laquelle $R_1$, R' et $R''_2$ sont définis comme précédement que, le cas échéant, soit l'on salifie, soit dans le cas où R' représente un radical aralkyle l'on soumet à l'action d'un agent de clivage, pour obtenir le composé de formule ($I_C$) :

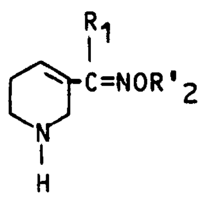

(I_C)

dans laquelle $R_1$ et $R'_2$ sont définis comme précédemment, que le cas échéant, l'on salifie.

4. Variante du procédé selon la revendication 3, caractérisée en ce que l'on soumet un composé de formule ($I'_A$) :

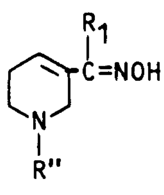

(I'_A)

dans laquelle R" représente un radical aralkyle renfermant jusqu'à 10 atomes de carbone et $R_1$ conserve sa signification précédente à l'action d'un agent de silylation, pour obtenir le composé de formule (VII) :

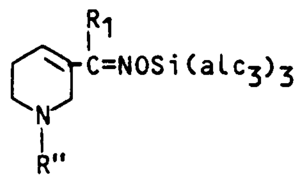

(VII)

dans laquelle $alc_3$ représente un radical alkyle renfermant de 1 à 8 atomes de carbone, que l'on soumet à l'action d'un agent de clivage, pour obtenir le composé de formule ($I_E$) :

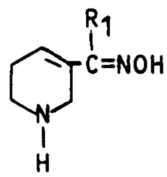

($I_E$)

dans laquelle $R_1$ est défini comme précédemment, que le cas échéant, l'on salifie.

**5.** Procédé selon la revendication 1, pour préparer les composés de formule (I) dans laquelle $R_1$ et $R_2$ ont la signification indiquée précédemment et R représente un radical hydroxyle caractérisé en ce que l'on soumet un composé de formule (VIII) :

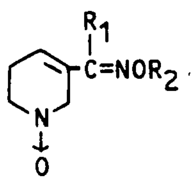

(VIII)

à l'action d'un agent réducteur pour obtenir un composé de formule ($I_F$) :

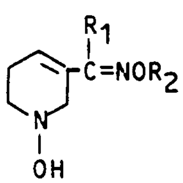

($I_F$)

dans laquelle $R_1$ et $R_2$ sont définis comme précédemment que, le cas échéant, l'on salifie.

**6.** Procédé selon l'une quelconque des revendications 1 à 5, caractérisé en ce que l'on utilise au départ un composé dans lequel $R_1$ représente un radical alkyle linéaire renfermant de 1 à 4 atomes de carbone.

**7.** Procédé selon l'une quelconque des revendications 1 à 5, caractérisé en ce que l'on utilise au départ un composé dans lequel $R_1$ représente un radical méthyle.

**8.** Procédé selon la revendication 3, caractérisé en ce que l'on utilise au départ un composé de formule (V) dans laquelle R' représente un radical alkyle linéaire ou ramifié, saturé ou insaturé renfermant de 1 à 4 atomes de carbone.

**9.** Procédé selon la revendication 3, caractérisé en ce que l'on utilise au départ un composé de formule (V) dans laquelle R' représente un radical méthyle, éthyle, propyle ou allyle.

**10.** Procédé selon l'une quelconque des revendications 1 à 3 et 5 à 9, caractérisé en ce que l'on utilise au départ un composé dans lequel $R'_2$ ou $R_2$ représente un atome d'hydrogène.

**11.** Procédé selon l'une quelconque des revendications 1 à 3 et 5 à 9, caractérisé en ce que l'on utilise au départ un composé dans lequel $R'_2$ ou $R_2$ représente un radical alkyle renfermant de 1 à 4 atomes de carbone.

**12.** Procédé selon l'une quelconque des revendications 1 à 3 et 5 à 9, caractérisé en ce que l'on utilise au départ un composé dans lequel $R'_2$ ou $R_2$ représente un radical méthyle.

**13.** Procédé selon la revendication 1, caractérisé en ce que l'on prépare des composés de formule (I) dans laquelle R représente un atome d'hydrogène ou un radical hydroxyle, ainsi que leurs sels d'addition avec les acides.

**14.** Procédé selon la revendication 1, caractérisé en ce que l'on prépare l'un quelconque des composés de formule (I) dont les noms suivent :
- le 1-méthyl 3-acétyl 1,2,5,6,-tétrahydropyridine O-méthyl oxime et son chlorhydrate,
- le 3-acétyl 1,2,5,6-tétrahydropyridine O-méthyl oxime et son chlorhydrate,
- le 1-N-hydroxyl 3-acétyl 1,2,5,6-tétrahydropyridine O-méthyl oxime.

**Revendications pour l'Etat contractant suivant : GR**

**1.** Procédé pour préparer les composés de formule (I) :

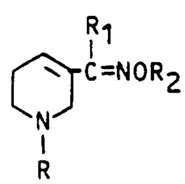

(I)

dans laquelle R représente un atome d'hydrogène, un radical hydroxyle, un radical alkyle linéaire, ramifié ou cyclique, saturé ou insaturé renfermant jusqu'à 8 atomes de carbone, ou R représente un radical aralkyle renfermant jusqu'à 10 atomes de carbone $R_1$ représente un radical alkyle linéaire, ramifié ou cyclique, saturé ou insaturé renfermant jusqu'à 8 atomes de carbone et $R_2$ représente un atome d'hydrogène ou un radical alkyle linéaire ou ramifié, saturé ou insaturé renfermant jusqu'à 8 atomes de carbone, un radical $COalc_1$ ou un radical $(CH_2)_2N(alc_2)_2$, $alc_1$ et $alc_2$ représentant un radical alkyle renfermant jusqu'à 8 atomes de carbone ainsi que leurs sels d'addition avec les acides à la condition que si R représente un radical alkyle, $R_2$ ne représente pas un atome d'hydrogène, caractérisé en ce que :

a) pour préparer les composés de formule (I) dans laquelle $R_1$ et $R_2$ ont la signification indiquée précédemment et R a la signification indiquée précédemment à l'exception de la valeur hydroxyle, l'on soumet un composé de formule (II) :

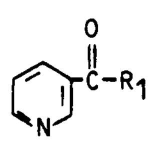

(II)

dans lequel $R_1$ conserve la même signification que précédemment, à l'action d'un composé de formule (III) :

$NH_2OR'_2$ (III)

ou de l'un de ses sels, dans laquelle $R'_2$ représente un atome d'hydrogène ou un radical alkyle

linéaire ou ramifié, saturé ou insaturé renfermant jusqu'à 8 atomes de carbone, pour obtenir le composé de formule (IV) :

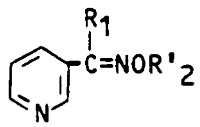

(IV)

que l'on soumet à l'action d'un halogénure d'alkyle de formule (V) :

R'-Hal (V)

dans laquelle Hal représente un atome d'halogène et R' représente un radical alkyle linéaire, ramifié ou cyclique, saturé ou insaturé renfermant jusqu'à 8 atomes de carbone éventuellement substitué par un radical carboxy libre ou estérifié ou R' représente un radical aralkyle renfermant jusqu'à 10 atomes de carbone, pour obtenir le composé de formule (VI) :

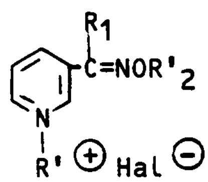

(VI)

que l'on soumet à l'action d'un agent d'hydrogénation, pour obtenir le composé de formule ($I_A$) :

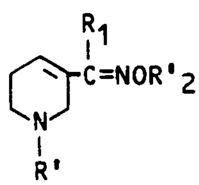

($I_A$)

dans laquelle R', $R_1$ et $R'_2$ sont définis comme précédemment que, le cas échéant, soit l'on salifie, soit l'on soumet dans le cas ou $R'_2$ représente un atome d'hydrogène, à l'action d'un composé de formule :

$R''_2$-Hal

dans laquelle Hal représente un atome d'halogène et $R''_2$ représente un radical $COalc_1$ ou un radical $-(CH_2)_2N(alc_2)_2$, $alc_1$ et $alc_2$ étant définis comme précédemment, pour obtenir le composé de formule ($I_B$) correspondant :

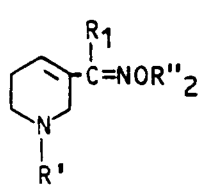

($I_B$)

dans laquelle $R_1$, R' et $R''_2$ sont définis comme précédement que, le cas échéant, l'on salifie, ou

dans le cas où R' représente un radical aralkyle l'on soumet le composé de formule ($I_A$) ou ($I_B$) à l'action d'un agent de clivage, pour obtenir le composé de formule ($I_C$) :

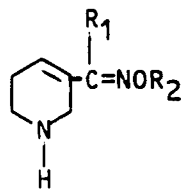

($I_C$)

dans laquelle $R_1$ et $R_2$ sont définis comme précédemment, que le cas échéant, l'on salifie.
b) pour préparer les composés de formule (I) dans laquelle $R_1$ et $R_2$ ont la signification indiquée précédemment et R représente un radical hydroxyle l'on soumet un composé de formule (VIII) :

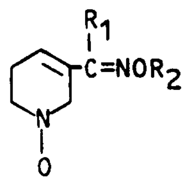

(VIII)

à l'action d'un agent réducteur pour obtenir un composé de formule ($I_F$) :

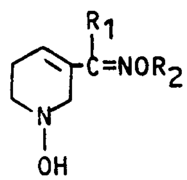

($I_F$)

dans laquelle $R_1$ et $R_2$ sont définis comme précédemment que, le cas échéant, l'on salifie.

**2.** Procédé selon la revendication 1, pour préparer les composés de formule (I) dans laquelle $R_1$ et $R_2$ ont la signification indiquée précédemment et R a la signification indiquée précédemment à l'exception de la valeur hydroxyle, caractérisé en ce que l'on soumet un composé de formule (II) :

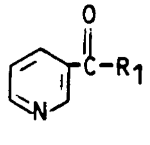

(II)

dans lequel $R_1$ conserve la même signification que précédemment, à l'action d'un composé de formule (III) :

$NH_2OR'_2$ (III)

ou de l'un de ses sels, dans laquelle $R'_2$ représente un atome d'hydrogène ou un radical alkyle linéaire ou ramifié, saturé ou insaturé renfermant jusqu'à 8 atomes de carbone, pour obtenir le composé de formule (IV) :

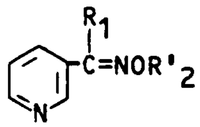

(IV)

que l'on soumet à l'action d'un halogénure d'alkyle de formule (V) :

R'-Hal (V)

dans laquelle Hal représente un atome d'halogène et R' représente un radical alkyle linéaire, ramifié ou cyclique, saturé ou insaturé renfermant jusqu'à 8 atomes de carbone éventuellement substitué par un radical carboxy libre ou estérifié ou R' représente un radical aralkyle renfermant jusqu'à 10 atomes de carbone, pour obtenir le composé de formule (VI) :

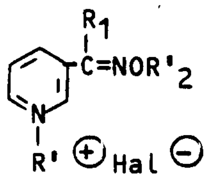

(VI)

que l'on soumet à l'action d'un agent d'hydrogénation, pour obtenir le composé de formule ($I_A$) :

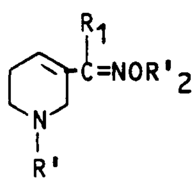

($I_A$)

dans laquelle R', $R_1$ et $R'_2$ sont définis comme précédemment que, le cas échéant, soit l'on salifie, soit l'on soumet dans le cas ou $R'_2$ représente un atome d'hydrogène, à l'action d'un composé de formule :

$R''_2$-Hal

dans laquelle Hal représente un atome d'halogène et $R''_2$ représente un radical $COalc_1$ ou un radical $-(CH_2)_2N(alc_2)_2$, $alc_1$ et $alc_2$ étant définis comme précédemment, pour obtenir le composé de formule ($I_B$) correspondant :

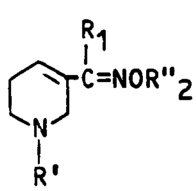

($I_B$)

dans laquelle $R_1$, R' et $R''_2$ sont définis comme précédement que, le cas échéant, soit l'on salifie, soit dans le cas où R' représente un radical aralkyle l'on soumet à l'action d'un agent de clivage, pour obtenir le composé de formule ($I_C$) :

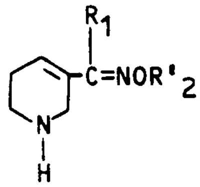

(I$_C$)

dans laquelle $R_1$ et $R'_2$ sont définis comme précédemment, que le cas échéant, l'on salifie.

3. Procédé selon la revendication 2, pour préparer les composés de formule (I) telle que définie à la revendication 1, répondant à la formule ($I_A$) :

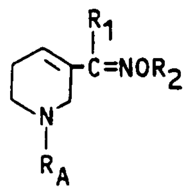

(I$_A$)

dans laquelle $R_1$ et $R_2$ sont définis comme à la revendication 1 et $R_A$ représente un atome d'hydrogène, un radical alkyle linéaire, ramifié ou cyclique, saturé ou insaturé renfermant jusqu'à 8 atomes de carbone, éventuellement substitué par un radical carboxy libre ou estérifié ou R représente un radical aralkyle renfermant jusqu'à 10 atomes de carbone ainsi que leurs sels d'addition avec les acides à la condition que si $R_A$ représente un radical alkyle, $R_2$ ne représente pas un atome d'hydrogène, caractérisé en ce que l'on soumet un composé de formule (II) :

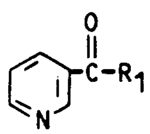

(II)

dans lequel $R_1$ conserve la même signification que précédemment, à l'action d'un composé de formule (III) :

$NH_2OR'_2$ (III)

ou de l'un de ses sels, dans laquelle $R'_2$ représente un atome d'hydrogène ou un radical alkyle linéaire ou ramifié, saturé ou insaturé renfermant jusqu'à 8 atomes de carbone, pour obtenir le composé de formule (IV) :

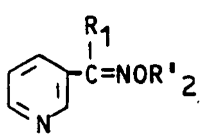

(IV)

que l'on soumet à l'action d'un halogénure d'alkyle de formule (V) :

R'-Hal (V)

dans laquelle Hal représente un atome d'halogène et R' représente un radical alkyle linéaire, ramifié ou cyclique, saturé ou insaturé renfermant jusqu'à 8 atomes de carbone éventuellement substitué par un radical carboxy libre ou estérifié ou R' représente un radical aralkyle renfermant jusqu'à 10 atomes de carbone, pour obtenir le composé de formule (VI) :

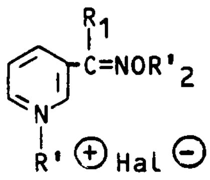

(VI)

que l'on soumet à l'action d'un agent d'hydrogénation, pour obtenir le composé de formule ($I_A$) :

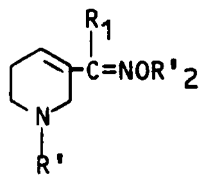

($I_A$)

dans laquelle R', $R_1$ et $R'_2$ sont définis comme précédemment que, le cas échéant, soit l'on salifie, soit l'on soumet dans le cas ou $R'_2$ représente un atome d'hydrogène, à l'action d'un composé de formule :

$R''_2$-Hal

dans laquelle Hal représente un atome d'halogène et $R''_2$ représente un radical $COalc_1$ ou un radical $-(CH_2)_2N(alc_2)_2$, $alc_1$ et $alc_2$ étant définis comme précédemment, pour obtenir le composé de formule ($I_B$) correspondant :

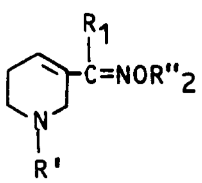

($I_B$)

dans laquelle $R_1$, R' et $R''_2$ sont définis comme précédement que, le cas échéant, soit l'on salifie, soit dans le cas où R' représente un radical aralkyle l'on soumet à l'action d'un agent de clivage, pour obtenir le composé de formule ($I_C$) :

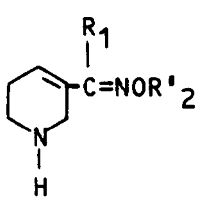

($I_C$)

dans laquelle $R_1$ et $R'_2$ sont définis comme précédemment, que le cas échéant, l'on salifie.

**4.** Variante du procédé selon la revendication 3, caractérisée en ce que l'on soumet un composé de formule ($I'_A$) :

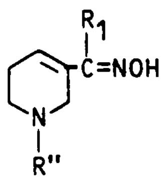

(I'$_A$)

dans laquelle R" représente un radical aralkyle renfermant jusqu'à 10 atomes de carbone et $R_1$ conserve sa signification précédente à l'action d'un agent de silylation, pour obtenir le composé de formule (VII) :

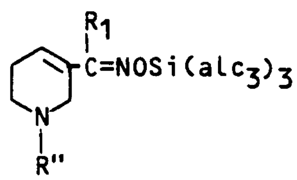

(VII)

dans laquelle $alc_3$ représente un radical alkyle renfermant de 1 à 8 atomes de carbone, que l'on soumet à l'action d'un agent de clivage, pour obtenir le composé de formule ($I_E$) :

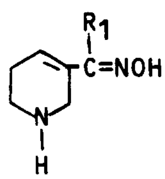

($I_E$)

dans laquelle $R_1$ est défini comme précédemment, que le cas échéant, l'on salifie.

**5.** Procédé selon la revendication 1, pour préparer les composés de formule (I) dans laquelle $R_1$ et $R_2$ ont la signification indiquée précédemment et R représente un radical hydroxyle caractérisé en ce que l'on soumet un composé de formule (VIII) :

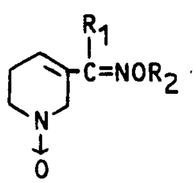

(VIII)

à l'action d'un agent réducteur pour obtenir un composé de formule ($I_F$) :

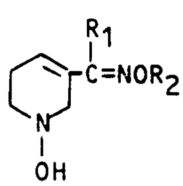

($I_F$)

dans laquelle $R_1$ et $R_2$ sont définis comme précédemment que, le cas échéant, l'on salifie.

**6.** Procédé selon l'une quelconque des revendications 1 à 5, caractérisé en ce que l'on utilise au départ

un composé dans lequel $R_1$ représente un radical alkyle linéaire renfermant de 1 à 4 atomes de carbone.

**7.** Procédé selon l'une quelconque des revendications 1 à 5, caractérisé en ce que l'on utilise au départ un composé dans lequel $R_1$ représente un radical méthyle.

**8.** Procédé selon la revendication 3, caractérisé en ce que l'on utilise au départ un composé de formule (V) dans laquelle R' représente un radical alkyle linéaire ou ramifié, saturé ou insaturé renfermant de 1 à 4 atomes de carbone.

**9.** Procédé selon la revendication 3, caractérisé en ce que l'on utilise au départ un composé de formule (V) dans laquelle R' représente un radical méthyle, éthyle, propyle ou allyle.

**10.** Procédé selon l'une quelconque des revendications 1 à 3 et 5 à 9, caractérisé en ce que l'on utilise au départ un composé dans lequel $R'_2$ ou $R_2$ représente un atome d'hydrogène.

**11.** Procédé selon l'une quelconque des revendications 1 à 3 et 5 à 9, caractérisé en ce que l'on utilise au départ un composé dans lequel $R'_2$ ou $R_2$ représente un radical alkyle renfermant de 1 à 4 atomes de carbone.

**12.** Procédé selon l'une quelconque des revendications 1 à 3 et 5 à 9, caractérisé en ce que l'on utilise au départ un composé dans lequel $R'_2$ ou $R_2$ représente un radical méthyle.

**13.** Procédé selon la revendication 1, caractérisé en ce que l'on prépare des composés de formule (I) dans laquelle R représente un atome d'hydrogène ou un radical hydroxyle, ainsi que leurs sels d'addition avec les acides.

**14.** Procédé selon la revendication 1, caractérisé en ce que l'on prépare l'un quelconque des composés de formule (I) dont les noms suivent :
- le 1-méthyl 3-acétyl 1,2,5,6,-tétrahydropyridine O-méthyl oxime et son chlorhydrate,
- le 3-acétyl 1,2,5,6-tétrahydropyridine O-méthyl oxime et son chlorhydrate,
- le 1-N-hydroxyl 3-acétyl 1,2,5,6-tétrahydropyridine O-méthyl oxime.

**15.** Procédé de préparation de compositions pharmaceutiques, caractérisé en ce que l'on met, à titre de principe actif, l'un au moins des composés de formule (I), telle que définie à la revendication 1, ou l'un au moins de leurs sels d'addition avec les acides pharmaceutiquement acceptables, sous une forme destinée à cet usage.

**16.** Procédé de préparation de compositions pharmaceutiques, caractérisé en ce que l'on met, à titre de principe actif, l'un au moins des composés de formule ($I_A$), telle que définie à la revendication 3, ou l'un au moins de leurs sels d'addition avec les acides pharmaceutiquement acceptables, sous une forme destinée à cet usage.

**17.** Procédé de préparation de compositions pharmaceutiques, caractérisé en ce que l'on met, à titre de principe actif, l'un au moins des composés de formule (I), telle que définie à la revendication 14, ou l'un au moins de leurs sels d'addition avec les acides pharmaceutiquement acceptables, sous une forme destinée à cet usage.

## Claims

## Claims for the following Contracting States : AT, BE, CH, DE, FR, GB, IT, LI, LU, NL, SE

**1.** The compounds of formula (I):

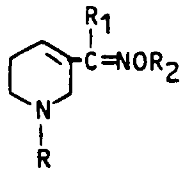

(I)

in which R represents a hydrogen atom, a hydroxyl radical, a saturated or unsaturated, linear, branched or cyclic alkyl radical containing up to 8 carbon atoms, or R represents an aralkyl radical containing up to 10 carbon atoms, $R_1$ represents a saturated or unsaturated, linear, branched or cyclic alkyl radical containing up to 8 carbon atoms and $R_2$ represents a hydrogen atom or a saturated or unsaturated, linear or branched alkyl radical containing up to 8 carbon atoms, a $COalk_1$ radical or a $(CH_2)_2N(alk_2)_2$ radical, $alk_1$ and $alk_2$ representing an alkyl radical containing up to 8 carbon atoms, as well as their addition salts with acids on condition that if R represents an alkyl radical, $R_2$ does not represent a hydrogen atom.

**2.** The compounds of formula (I) as defined in claim 1, in which $R_1$ represents a linear alkyl radical containing 1 to 4 carbon atoms as well as their addition salts with acids.

**3.** The compounds of formula (I) as defined in claim 2, in which $R_1$ represents a methyl radical, as well as their addition salts with acids.

**4.** The compounds of formula (I) as defined in any one of claims 1 to 3 in which R represents a hydrogen atom or a hydroxyl radical, as well as their addition salts with acids.

**5.** The compounds of formula (I) as defined in any one of claims 1 to 3 in which R represents a saturated or unsaturated, linear or branched alkyl radical containing 1 to 4 carbon atoms, as well as their addition salts with acids.

**6.** The compounds of formula (I) as defined in claim 5, in which R represents a methyl, ethyl, propyl or allyl radical, as well as their addition salts with acids.

**7.** The compounds of formula (I) as defined in any one of claims 1 to 6, in which $R_2$ represents a hydrogen atom, as well as their addition salts with acids.

**8.** The compounds of formula (I) as defined in any one of claims 1 to 6, in which $R_2$ represents an alkyl radical containing 1 to 4 carbon atoms and in particular a methyl radical.

**9.** The compounds of formula (I) as defined in claim 1 of which the names follow:
- 1-methyl-3-acetyl-1,2,5,6-tetrahydropyridine-O-methyl oxime and its hydrochloride,
- 3-acetyl-1,2,5,6-tetrahydropyridine-O-methyl oxime and its hydrochloride,
- 1-N-hydroxyl-3-acetyl-1,2,5,6-tetrahydropyridine-O-methyl oxime.

**10.** Preparation process for compounds of formula (I) in which $R_1$ and $R_2$ have the meaning indicated previously and R has the meaning indicated previously with the exception of the hydroxyl value, characterized in that a compound of formula (II):

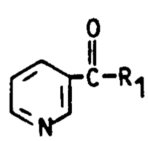

(II)

in which $R_1$ keeps the same meaning as previously, is subjected to the action of a compound of formula (III):

$NH_2OR'_2$ (III)

or of one of its salts, in which $R'_2$ represents a hydrogen atom or a saturated or unsaturated, linear or branched alkyl radical containing up to 8 carbon atoms, in order to obtain the compound of formula (IV):

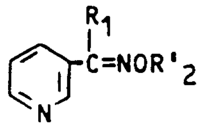

(IV)

which is subjected to the action of an alkyl halide of formula (V):

R'-Hal (V)

in which Hal represents a halogen atom and R' represents a saturated or unsaturated, linear, branched or cyclic alkyl radical containing up to 8 carbon atoms, optionally substituted by a free or esterified carboxy radical or R' represents an aralkyl radical containing up to 10 carbon atoms, in order to obtain the compound of formula (VI):

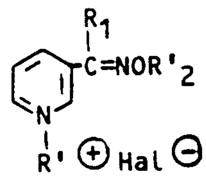

(VI)

which is subjected to the action of a hydrogenation agent, in order to obtain the compound of formula ($I_A$):

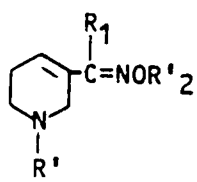

($I_A$)

in which R', $R_1$ and $R'_2$ are defined as previously, which, if appropriate, either is salified, or is subjected, in the case where $R'_2$ represents a hydrogen atom, to the action of a compound of formula:

$R''_2$-Hal

in which Hal represents a halogen atom and $R''_2$ represents a COalk$_1$ radical or a $-(CH_2)_2N(alk_2)_2$ radical, alk$_1$ and alk$_2$ being defined as previously, in order to obtain the corresponding compound of formula ($I_B$):

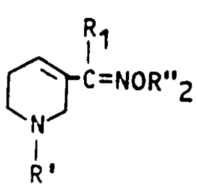

($I_B$)

in which $R_1$, R' and $R''_2$ are defined as previously, which, if appropriate, is salified, or in the case where R' represents an aralkyl radical, the compound of formula ($I_A$) or ($I_B$) is subjected to the action of a cleavage agent, in order to obtain the compound of formula ($I_C$):

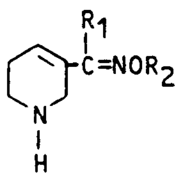

($I_C$)

in which $R_1$ and $R_2$ are defined as previously, which if appropriate is salified.

**11.** Variant of the process according to claim 10, characterized in that a compound of formula ($I'_A$):

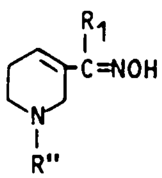

($I'_A$)

in which R" represents an aralkyl radical containing up to 10 carbon atoms and $R_1$ keeps its previous meaning, is subjected to the action of a silylation agent, in order to obtain the compound of formula (VII):

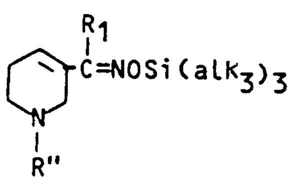

(VII)

in which $alk_3$ represents an alkyl radical containing 1 to 8 carbon atoms, which is subjected to the action of a cleavage agent, in order to obtain the compound of formula ($I_E$):

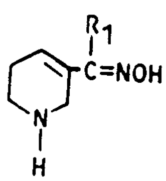

($I_E$)

in which $R_1$ is defined as previously, which if appropriate is salified.

**12.** Preparation process for compounds of formula (I) in which $R_1$ and $R_2$ have the meaning indicated previously and R represents a hydroxyl radical, characterized in that a compound of formula (VIII):

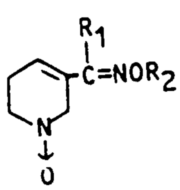

(VIII)

is subjected to the action of a reducing agent in order to obtain a compound of formula ($I_F$):

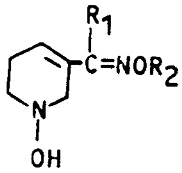

($I_F$)

in which $R_1$ and $R_2$ are defined as previously, which if appropriate is salified.

**13.** As medicaments, the compounds of formula (I) as defined in any one of claims 1 to 9, as well as their addition salts with pharmaceutically acceptable acids.

**14.** The pharmaceutical compositions containing as active ingredient at least one medicament defined in claim 13.

**Claims for the following Contracting State : ES**

**1.** Process for preparing the compounds of formula (I):

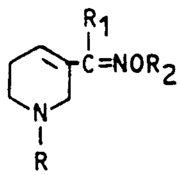

(I)

in which R represents a hydrogen atom, a hydroxyl radical, a saturated or unsaturated, linear, branched or cyclic alkyl radical containing up to 8 carbon atoms, or R represents an aralkyl radical containing up to 10 carbon atoms, $R_1$ represents a saturated or unsaturated, linear, branched or cyclic alkyl radical containing up to 8 carbon atoms and $R_2$ represents a hydrogen atom or a saturated or unsaturated, linear or branched alkyl radical containing up to 8 carbon atoms, a $COalk_1$ radical or a $(CH_2)_2N(alk_2)_2$ radical, $alk_1$ and $alk_2$ representing an alkyl radical containing up to 8 carbon atoms, as well as their addition salts with acids on condition that if R represents an alkyl radical, $R_2$ does not represent a hydrogen atom, characterized in that:

a) to prepare the compounds of formula (I) in which $R_1$ and $R_2$ have the meaning indicated previously and R has the meaning indicated previously with the exception of the hydroxyl value, a compound of formula (II):

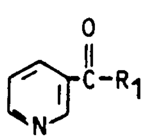

(II)

in which $R_1$ keeps the same meaning as previously, is subjected to the action of a compound of formula (III):

$NH_2OR'_2$ (III)

or of one of its salts, in which $R'_2$ represents a hydrogen atom or a saturated or unsaturated, linear or branched alkyl radical containing up to 8 carbon atoms, in order to obtain the compound of formula (IV):

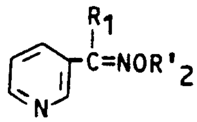

(IV)

which is subjected to the action of an alkyl halide of formula (V):

R'-Hal (V)

in which Hal represents a halogen atom and R' represents a saturated or unsaturated, linear, branched or cyclic alkyl radical containing up to 8 carbon atoms optionally substituted by a free or esterified carboxy radical or R' represents an aralkyl radical containing up to 10 carbon atoms, in order to obtain the compound of formula (VI):

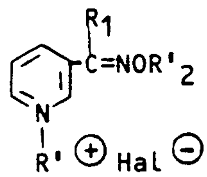

(VI)

which is subjected to the action of a hydrogenation agent, in order to obtain the compound of formula ($I_A$):

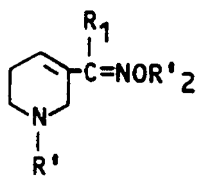

($I_A$)

in which R', $R_1$ and $R'_2$ are defined as previously, which if appropriate, either is salified, or is subjected, in the case where $R'_2$ represents a hydrogen atom, to the action of a compound of formula:

$R''_2$-Hal

in which Hal represents a halogen atom and $R''_2$ represents a $COalk_1$ radical or a $-(CH_2)_2N(alk_2)_2$ radical, $alk_1$ and $alk_2$ being defined as previously, in order to obtain the corresponding compound of formula ($I_B$):

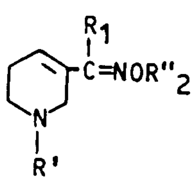

($I_B$)

in which $R_1$, R' and $R''_2$ are defined as previously, which if appropriate is salified, or in the case where R' represents an aralkyl radical, the compound of formula ($I_A$) or ($I_B$) is subjected to the action of a cleavage agent, in order to obtain the compound of formula ($I_C$):

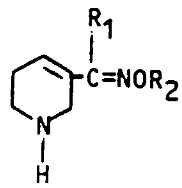

($I_C$)

in which $R_1$ and $R_2$ are defined as previously, which if appropriate is salified;

b) to prepare the compounds of formula (I) in which $R_1$ and $R_2$ have the meaning indicated previously and R represents a hydroxyl radical, a compound of formula (VIII):

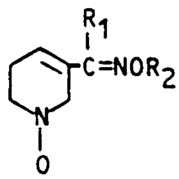

(VIII)

is subjected to the action of a reducing agent in order to obtain a compound of formula ($I_F$):

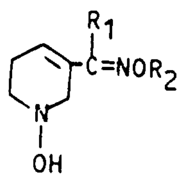

($I_F$)

in which $R_1$ and $R_2$ are defined as previously, which if appropriate is salified.

**2.** Process according to claim 1, for preparing the compounds of formula (I) in which $R_1$ and $R_2$ have the meaning indicated previously and R has the meaning indicated previously with the exception of the hydroxyl value, characterized in that a compound of formula (II):

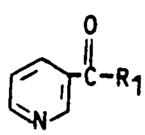

(II)

in which $R_1$ keeps the same meaning as previously, is subjected to the action of a compound of formula (III):

$NH_2OR'_2$ (III)

or of one of its salts, in which $R'_2$ represents a hydrogen atom or a saturated or unsaturated, linear or branched alkyl radical containing up to 8 carbon atoms, in order to obtain the compound of formula (IV):

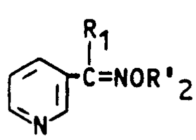

(IV)

which is subjected to the action of an alkyl halide of formula (V):

R'-Hal (V)

in which Hal represents a halogen atom and R' represents a saturated or unsaturated, linear, branched or cyclic alkyl radical containing up to 8 carbon atoms optionally substituted by a free or esterified carboxy radical or R' represents an aralkyl radical containing up to 10 carbon atoms, in order to obtain the compound of formula (VI):

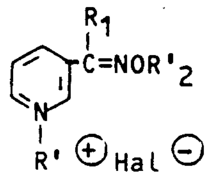

(VI)

which is subjected to the action of a hydrogenation agent, in order to obtain the compound of formula ($I_A$):

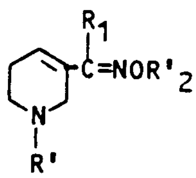

($I_A$)

in which R', $R_1$ and $R'_2$ are defined as previously, which if appropriate either is salified, or is subjected, in the case where $R'_2$ represents a hydrogen atom, to the action of a compound of formula:

$R''_2$-Hal

in which Hal represents a halogen atom and $R''_2$ represents a $COalk_1$ radical or a $-(CH_2)_2N(alk_2)_2$ radical, $alk_1$ and $alk_2$ being defined as previously, in order to obtain the corresponding compound of formula ($I_B$):

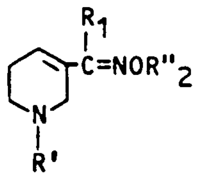

($I_B$)

in which $R_1$, R' and $R''_2$ are defined as previously, which if appropriate either is salified, or in the case where R' represents an aralkyl radical, is subjected to the action of a cleavage agent, in order to obtain the compound of formula ($I_C$):

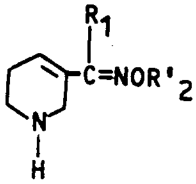

($I_C$)

in which $R_1$ and $R'_2$ are defined as previously, which if appropriated is salified.

3. Process according to claim 2, for preparing the compounds of formula (I) as defined in claim 1, corresponding to the formula ($I_A$):

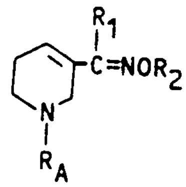

($I_A$)

in which $R_1$ and $R_2$ are defined as in claim 1 and $R_A$ represents a hydrogen atom, a saturated or unsaturated, linear, branched or cyclic alkyl radical containing up to 8 carbon atoms, optionally substituted by a free or esterified carboxy radical or R represents an aralkyl radical containing up to 10 carbon atoms, as well as their addition salts with acids, on condition that if $R_A$ represents an alkyl radical, $R_2$ does not represent a hydrogen atom, characterized in that a compound of formula (II):

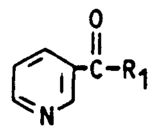

(II)

in which $R_1$ keeps the same meaning as previously, is subjected to the action of a compound of formula (III):

$NH_2OR'_2$ (III)

or of one of its salts, in which $R'_2$ represents a hydrogen atom or a saturated or unsaturated, linear or branched alkyl radical containing up to 8 carbon atoms, in order to obtain the compound of formula (IV):

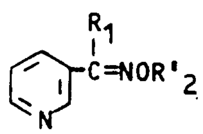

(IV)

which is subjected to the action of an alkyl halide of formula (V):

R'-Hal (V)

in which Hal represents a halogen atom and R' represents a saturated or unsaturated, linear branched or cyclic alkyl radical containing up to 8 carbon atoms, optionally substituted by a free or esterified carboxy radical or R' represents an aralkyl radical containing up to 10 carbon atoms, in order to obtain the compound of formula (VI):

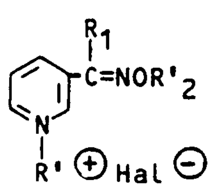

(VI)

which is subjected to the action of a hydrogenation agent, in order to obtain the compound of formula ($I_A$):

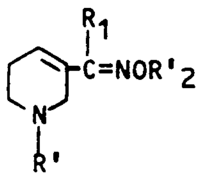

($I_A$)

in which R', $R_1$ and $R'_2$ are defined as previously, which if appropriate either is salified, or is subjected, in the case where $R'_2$ represents a hydrogen atom, to the action of a compound of formula:

$R''_2$-Hal

in which Hal represents a halogen atom and $R''_2$ represents a $COalk_1$ radical or a $-(CH_2)_2N(alk_2)_2$ radical, $alk_1$ and $alk_2$ being defined as previously, in order to obtain the corresponding compound of formula ($I_B$):

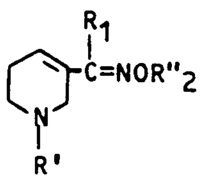

($I_B$)

in which $R_1$, R' and $R''_2$ are defined as previously, which if appropriate either is salified, or in the case where R' represents an aralkyl radical, is subjected to the action of a cleavage agent, in order to obtain the compound of formula ($I_C$):

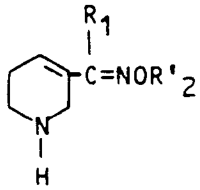

($I_C$)

in which $R_1$ and $R'_2$ are defined as previously, which if appropriate is salified.

**4.** Variant of the process according to claim 3, characterized in that a compound of formula ($I'_A$):

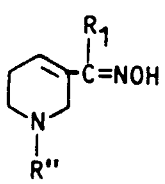

($I'_A$)

in which R" represents an aralkyl radical containing up to 10 carbon atoms and $R_1$ keeps its previous meaning, is subjected to the action of a silylation agent, in order to obtain the compound of formula (VII):

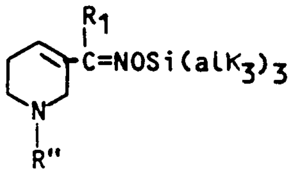

(VII)

in which $alk_3$ represents an alkyl radical containing 1 to 8 carbon atoms, which is subjected to the action of a cleavage agent, in order to obtain the compound of formula ($I_E$):

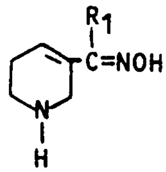

($I_E$)

in which $R_1$ is defined as previously, which if appropriate is salified.

**5.** Process according to claim 1, for preparing the compounds of formula (I) in which $R_1$ and $R_2$ have the meaning indicated previously and R represents a hydroxyl radical, characterized in that a compound of formula (VIII):

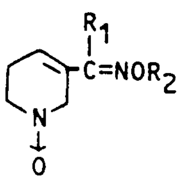

(VIII)

is subjected to the action of a reducing agent in order to obtain a compound of formula ($I_F$):

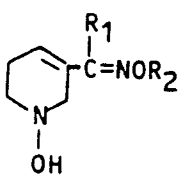

($I_F$)

in which $R_1$ and $R_2$ are defined as previously, which if appropriate is salified.

**6.** Process according to any one of claims 1 to 5, characterized in that a compound in which $R_1$ represents a linear alkyl radical containing 1 to 4 carbon atoms is used at the start.

**7.** Process according to any one of claims 1 to 5, characterized in that a compound in which $R_1$ represents a methyl radical is used at the start.

**8.** Process according to claim 3, characterized in that a compound of formula (V) in which R' represents a saturated or unsaturated, linear or branched alkyl radical containing 1 to 4 carbon atoms is used at the start.

**9.** Process according to claim 3, characterized in that a compound of formula (V) in which R' represents a methyl, ethyl, propyl or allyl radical is used at the start.

**10.** Process according to any one of claims 1 to 3 and 5 to 9, characterized in that a compound in which $R'_2$ or $R_2$ represents a hydrogen atom is used at the start.

**11.** Process according to any one of claims 1 to 3 and 5 to 9, characterized in that a compound in which $R'_2$ or $R_2$ represents an alkyl radical containing 1 to 4 carbon atoms is used at the start.

**12.** Process according to any one of claims 1 to 3 and 5 to 9, characterized in that a compound in which $R'_2$ or $R_2$ represents a methyl radical is used at the start.

**13.** Process according to claim 1, characterized in that compounds of formula (I) are prepared in which R represents a hydrogen atom or a hydroxyl radical, as well as their addition salts with acids.

**14.** Process according to claim 1, characterized in that any one of the compounds of formula (I) are prepared, of which the names follow:
- 1-methyl-3-acetyl-1,2,5,6-tetrahydropyridine-O-methyl oxime and its hydrochloride,
- 3-acetyl-1,2,5,6-tetrahydropyridine-O-methyl oxime and its hydrochloride,
- 1-N-hydroxyl-3-acetyl-1,2,5,6-tetrahydropyridine-O-methyl oxime.

**Claims for the following Contracting State : GR**

**1.** Process for preparing the compounds of formula (I):

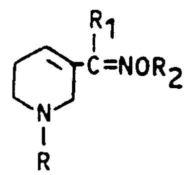

(I)

in which R represents a hydrogen atom, a hydroxyl radical, a saturated or unsaturated, linear, branched or cyclic alkyl radical containing up to 8 carbon atoms, or R represents an aralkyl radical containing up to 10 carbon atoms, $R_1$ represents a saturated or unsaturated, linear, branched or cyclic alkyl radical containing up to 8 carbon atoms and $R_2$ represents a hydrogen atom or a saturated or unsaturated, linear or branched alkyl radical containing up to 8 carbon atoms, a $COalk_1$ radical or a $(CH_2)_2N(alk_2)_2$ radical, $alk_1$ and $alk_2$ representing an alkyl radical containing up to 8 carbon atoms, as well as their addition salts with acids on condition that if R represents an alkyl radical, $R_2$ does not represent a hydrogen atom, characterized in that:

a) to prepare the compounds of formula (I) in which $R_1$ and $R_2$ have the meaning indicated previously and R has the meaning indicated previously with the exception of the hydroxyl value, a compound of formula (II):

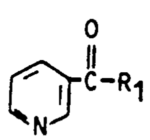

(II)

in which $R_1$ keeps the same meaning as previously, is subjected to the action of a compound of formula (III):

$NH_2OR'_2$ (III)

or of one of its salts, in which $R'_2$ represents a hydrogen atom or a saturated or unsaturated, linear or branched alkyl radical containing up to 8 carbon atoms, in order to obtain the compound of formula (IV):

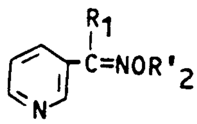

(IV)

which is subjected to the action of an alkyl halide of formula (V):

R'-Hal (V)

in which Hal represents a halogen atom and R' represents a saturated or unsaturated, linear, branched or cyclic alkyl radical containing up to 8 carbon atoms optionally substituted by a free or esterified carboxy radical or R' represents an aralkyl radical containing up to 10 carbon atoms, in order to obtain the compound of formula (VI):

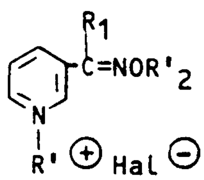

(VI)

which is subjected to the action of a hydrogenation agent, in order to obtain the compound of formula ($I_A$):

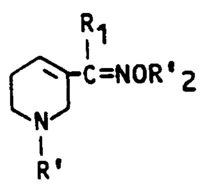

($I_A$)

in which R', $R_1$ and $R'_2$ are defined as previously, which if appropriate, either is salified, or is subjected, in the case where $R'_2$ represents a hydrogen atom, to the action of a compound of formula:

$R''_2$-Hal

in which Hal represents a halogen atom and $R''_2$ represents a $COalk_1$ radical or a $-(CH_2)_2N(alk_2)_2$ radical, $alk_1$ and $alk_2$ being defined as previously, in order to obtain the corresponding compound of formula ($I_B$):

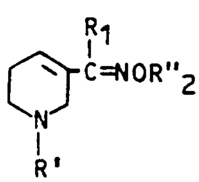

($I_B$)

in which $R_1$, R' and $R''_2$ are defined as previously, which if appropriate is salified, or in the case where R' represents an aralkyl radical, the compound of formula ($I_A$) or ($I_B$) is subjected to the action of a cleavage agent, in order to obtain the compound of formula ($I_C$):

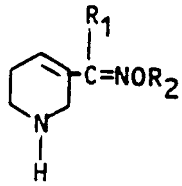

($I_C$)

in which $R_1$ and $R_2$ are defined as previously, which if appropriate is salified;

b) to prepare the compounds of formula (I) in which $R_1$ and $R_2$ have the meaning indicated previously and R represents a hydroxyl radical, a compound of formula (VIII):

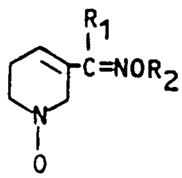

(VIII)

is subjected to the action of a reducing agent in order to obtain a compound of formula ($I_F$):

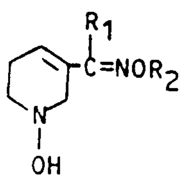

($I_F$)

in which $R_1$ and $R_2$ are defined as previously, which if appropriate is salified.

**2.** Process according to claim 1, for preparing the compounds of formula (I) in which $R_1$ and $R_2$ have the meaning indicated previously and R has the meaning indicated previously with the exception of the hydroxyl value, characterized in that a compound of formula (II):

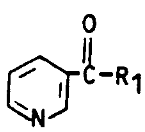

(II)

in which $R_1$ keeps the same meaning as previously, is subjected to the action of a compound of formula (III):

$NH_2OR'_2$ (III)

or of one of its salts, in which $R'_2$ represents a hydrogen atom or a saturated or unsaturated, linear or branched alkyl radical containing up to 8 carbon atoms, in order to obtain the compound of formula (IV):

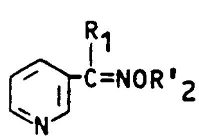

(IV)

which is subjected to the action of an alkyl halide of formula (V):

R'-Hal (V)

in which Hal represents a halogen atom and R' represents a saturated or unsaturated, linear, branched or cyclic alkyl radical containing up to 8 carbon atoms optionally substituted by a free or esterified carboxy radical or R' represents an aralkyl radical containing up to 10 carbon atoms, in order to obtain the compound of formula (VI):

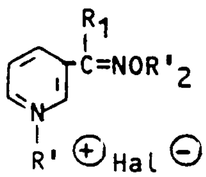

(VI)

which is subjected to the action of a hydrogenation agent, in order to obtain the compound of formula ($I_A$):

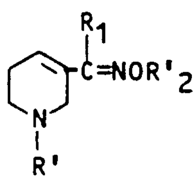

($I_A$)

in which R', $R_1$ and $R'_2$ are defined as previously, which if appropriate either is salified, or is subjected, in the case where $R'_2$ represents a hydrogen atom, to the action of a compound of formula:

$R''_2$-Hal

in which Hal represents a halogen atom and $R''_2$ represents a $COalk_1$ radical or a $-(CH_2)_2N(alk_2)_2$ radical, $alk_1$ and $alk_2$ being defined as previously, in order to obtain the corresponding compound of formula ($I_B$):

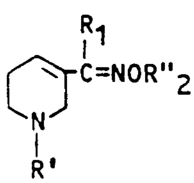

($I_B$)

in which $R_1$, R' and $R''_2$ are defined as previously, which if appropriate either is salified, or in the case where R' represents an aralkyl radical, is subjected to the action of a cleavage agent, in order to obtain the compound of formula ($I_C$):

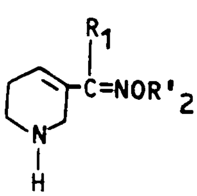

($I_C$)

in which $R_1$ and $R'_2$ are defined as previously, which if appropriated is salified.

**3.** Process according to claim 2, for preparing the compounds of formula (I) as defined in claim 1, corresponding to the formula ($I_A$):

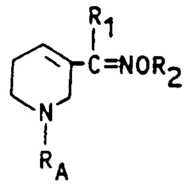

($I_A$)

in which $R_1$ and $R_2$ are defined as in claim 1 and $R_A$ represents a hydrogen atom, a saturated or unsaturated, linear, branched or cyclic alkyl radical containing up to 8 carbon atoms, optionally substituted by a free or esterified carboxy radical or R represents an aralkyl radical containing up to 10 carbon atoms, as well as their addition salts with acids, on condition that if $R_A$ represents an alkyl radical, $R_2$ does not represent a hydrogen atom, characterized in that a compound of formula (II):

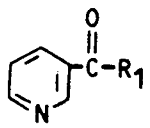

(II)

in which $R_1$ keeps the same meaning as previously, is subjected to the action of a compound of formula (III):

$NH_2OR'_2$ (III)

or of one of its salts, in which $R'_2$ represents a hydrogen atom or a saturated or unsaturated, linear or branched alkyl radical containing up to 8 carbon atoms, in order to obtain the compound of formula (IV):

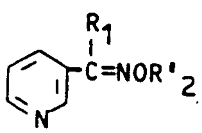

(IV)

which is subjected to the action of an alkyl halide of formula (V):

R'-Hal (V)

in which Hal represents a halogen atom and R' represents a saturated or unsaturated, linear branched or cyclic alkyl radical containing up to 8 carbon atoms, optionally substituted by a free or esterified carboxy radical or R' represents an aralkyl radical containing up to 10 carbon atoms, in order to obtain the compound of formula (VI):

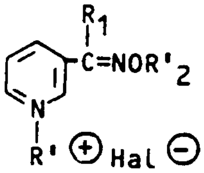

(VI)

which is subjected to the action of a hydrogenation agent, in order to obtain the compound of formula ($I_A$):

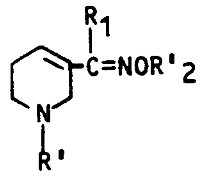

($I_A$)

in which R', $R_1$ and $R'_2$ are defined as previously, which if appropriate either is salified, or is subjected, in the case where $R'_2$ represents a hydrogen atom, to the action of a compound of formula:

$R''_2$-Hal

in which Hal represents a halogen atom and $R''_2$ represents a $COalk_1$ radical or a $-(CH_2)_2N(alk_2)_2$ radical, $alk_1$ and $alk_2$ being defined as previously, in order to obtain the corresponding compound of formula ($I_B$):

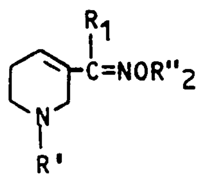

($I_B$)

in which $R_1$, R' and $R''_2$ are defined as previously, which if appropriate either is salified, or in the case where R' represents an aralkyl radical, is subjected to the action of a cleavage agent, in order to obtain the compound of formula ($I_C$):

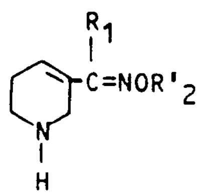

($I_C$)

in which $R_1$ and $R'_2$ are defined as previously, which if appropriate is salified.

**4.** Variant of the process according to claim 3, characterized in that a compound of formula ($I'_A$):

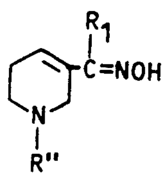

($I'_A$)

in which R" represents an aralkyl radical containing up to 10 carbon atoms and $R_1$ keeps its previous meaning, is subjected to the action of a silylation agent, in order to obtain the compound of formula (VII):

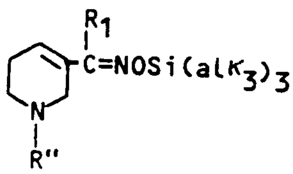

(VII)

in which $alk_3$ represents an alkyl radical containing 1 to 8 carbon atoms, which is subjected to the action of a cleavage agent, in order to obtain the compound of formula ($I_E$):

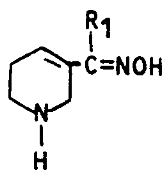

($I_E$)

in which $R_1$ is defined as previously, which if appropriate is salified.

**5.** Process according to claim 1, for preparing the compounds of formula (I) in which $R_1$ and $R_2$ have the meaning indicated previously and R represents a hydroxyl radical, characterized in that a compound of formula (VIII):

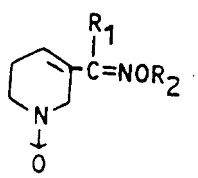

(VIII)

is subjected to the action of a reducing agent in order to obtain a compound of formula ($I_F$):

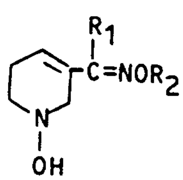

($I_F$)

in which $R_1$ and $R_2$ are defined as previously, which if appropriate is salified.

**6.** Process according to any one of claims 1 to 5, characterized in that a compound in which $R_1$ represents a linear alkyl radical containing 1 to 4 carbon atoms is used at the start.

**7.** Process according to any one of claims 1 to 5, characterized in that a compound in which $R_1$ represents a methyl radical is used at the start.

**8.** Process according to claim 3, characterized in that a compound of formula (V) in which R' represents a saturated or unsaturated, linear or branched alkyl radical containing 1 to 4 carbon atoms is used at the start.

**9.** Process according to claim 3, characterized in that a compound of formula (V) in which R' represents a methyl, ethyl, propyl or allyl radical is used at the start.

**10.** Process according to any one of claims 1 to 3 and 5 to 9, characterized in that a compound in which $R'_2$ or $R_2$ represents a hydrogen atom is used at the start.

**11.** Process according to any one of claims 1 to 3 and 5 to 9, characterized in that a compound in which $R'_2$ or $R_2$ represents an alkyl radical containing 1 to 4 carbon atoms is used at the start.

**12.** Process according to any one of claims 1 to 3 and 5 to 9, characterized in that a compound in which $R'_2$ or $R_2$ represents a methyl radical is used at the start.

**13.** Process according to claim 1, characterized in that compounds of formula (I) are prepared in which R represents a hydrogen atom or a hydroxyl radical, as well as their addition salts with acids.

**14.** Process according to claim 1, characterized in that any one of the compounds of formula (I) are prepared, of which the names follow:
- 1-methyl-3-acetyl-1,2,5,6-tetrahydropyridine-O-methyl oxime and its hydrochloride,
- 3-acetyl-1,2,5,6-tetrahydropyridine-O-methyl oxime and its hydrochloride,
- 1-N-hydroxyl-3-acetyl-1,2,5,6-tetrahydropyridine-O-methyl oxime.

**15.** Preparation process for pharmaceutical compositions, characterized in that at least one of the compounds of formula (I), as defined in claim 1, or at least one of their addition salts with pharmaceutically acceptable acids, is used as active ingredient in a form intended for this use.

**16.** Preparation process for pharmaceutical compositions, characterized in that at least one of the compounds of formula ($I_A$), as defined in claim 3, or at least one of their addition salts with pharmaceutically acceptable acids, is used as active ingredient in a form intended for this use.

**17.** Preparation process for pharmaceutical compositions, characterized in that at least one of the compounds of formula (I), as defined in claim 14, or at least one of their addition salts with pharmaceutically acceptable acids, is used as active ingredient in a form intended for this use.

**Patentansprüche**
**Patentansprüche für folgende Vertragsstaaten : AT, BE, CH, DE, FR, GB, IT, LI, LU, NL, SE**

**1.** Verbindungen der Formel (I)

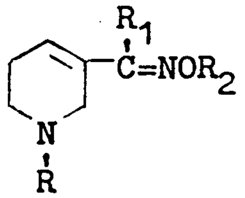

(I)

worin R ein Wasserstoffatom, einen Hydroxylrest, einen gesättigten oder ungesättigten, linearen, verzweigten oder cyclischen Alkylrest mit bis zu 8 Kohlenstoffatomen wiedergibt oder R einen Aralkylrest mit bis zu 10 Kohlenstoffatomen bedeutet, $R_1$ für einen gesättigten oder ungesättigten, linearen, verzweigten oder cyclischen Alkylrest mit bis zu 8 Kohlenstoffatomen steht und $R_2$ ein Wasserstoffatom oder einen gesättigten oder ungesättigten, linearen oder verzweigten Alkylrest mit bis zu 8 Kohlenstoffatomen, einen Rest $COalc_1$ oder einen Rest $(CH_2)_2N(alc_2)_2$ bedeutet, wobei $alc_1$ und $alc_2$ einen Alkylrest mit bis zu 8 Kohlenstoffatomen wiedergeben, sowie deren Additionssalze mit Säuren, mit der Maßgabe, daß, wenn R für einen Alkylrest steht, $R_2$ kein Wasserstoffatom bedeutet.

**2.** Verbindungen der Formel (I) gemäß Anspruch 1, worin $R_1$ für einen linearen Alkylrest mit 1 bis 4 Kohlenstoffatomen steht, sowie deren Additionssalze mit Säuren.

**3.** Verbindungen der Formel (I) gemäß Anspruch 2, worin $R_1$ einen Methylrest bedeutet, sowie deren Additionssalze mit Säuren.

4. Verbindungen der Formel (I) gemäß einem der Ansprüche 1 bis 3, worin R ein Wasserstoffatom oder eine Hydroxylgruppe bedeutet, sowie deren Additionssalze mit Säuren.

5. Verbindungen der Formel (I) gemäß einem der Ansprüche 1 bis 3, worin R einen gesättigten oder ungesättigten, linearen oder verzweigten Alkylrest mit 1 bis 4 Kohlenstoffatomen bedeutet, sowie deren Additionssalze mit Säuren.

6. Verbindungen der Formel (I) gemäß Anspruch 5, worin R einen Methyl-, Ethyl-, Propyl- oder Allylrest bedeutet, sowie deren Additionssalze mit Säuren.

7. Verbindungen der Formel (I) gemäß einem der Ansprüche 1 bis 6, worin $R_2$ ein Wasserstoffatom bedeutet, sowie deren Additionssalze mit Säuren.

8. Verbindungen der Formel (I) gemäß einem der Ansprüche 1 bis 6, worin $R_2$ einen Alkylrest mit 1 bis 4 Kohlenstoffatomen und insbesondere einen Methylrest bedeutet.

9. Verbindungen der Formel (I) gemäß Anspruch 1 mit den folgenden Bezeichnungen:
   1-Methyl-3-acetyl-1,2,5,6-tetrahydropyridin-O-methyloxim und dessen Hydrochlorid,
   3-Acetyl-1,2,5,6-tetrahydropyridin-O-methyloxim und dessen Hydrochlorid,
   1-N-Hydroxyl-3-acetyl-1,2,5,6-tetrahydropyridin-O-methyloxim.

10. Verfahren zur Herstellung der Verbindungen der Formel (I), worin $R_1$ und $R_2$ die vorstehend angegebene Bedeutung besitzen und R die vorstehend angegebene Bedeutung mit Ausnahme von Hydroxyl wiedergibt, dadurch gekennzeichnet, daß man eine Verbindung der Formel (II)

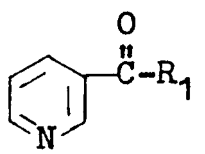

(II)

worin $R_1$ die vorstehend angegebene Bedeutung besitzt, der Einwirkung einer Verbindung der Formel (III)

$NH_2OR'_2$ (III)

oder derjenigen eines ihrer Salze, worin $R'_2$ ein Wasserstoffatom oder einen gesättigten oder ungesättigten, linearen oder verzweigten Alkylrest mit bis zu 8 Kohlenstoffatomen bedeutet, unterzieht, um zu der Verbindung der Formel (IV)

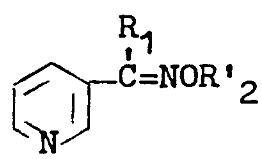

(IV)

zu gelangen, die man der Einwirkung eines Alkylhalogenids der Formel (V)

R'-Hal (V)

unterzieht, worin Hal für ein Halogenatom steht und R' einen gesättigten oder ungesättigten, linearen, verzweigten oder cyclischen Alkylrest mit bis zu 8 Kohlenstoffatomen, der gegebenenfalls durch eine freie oder veresterte Carboxygruppe substituiert ist, bedeutet oder R' für einen Aralkylrest mit bis zu 10 Kohlenstoffatomen steht, um zu der Verbindung der Formel (VI)

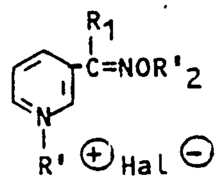

(VI)

Zu gelangen, die man der Einwirkung eines Hydrierungsmittels unterzieht, um die Verbindung der Formel ($I_A$)

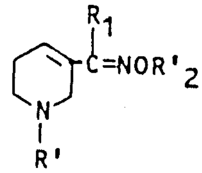

($I_A$)

worin R', $R_1$ und $R'_2$ wie vorstehend definiert sind, zu erhalten, die man gegebenenfalls entweder in ein Salz überführt oder, wenn $R'_2$ ein Wasserstoffatom bedeutet, der Einwirkung einer Verbindung der Formel

$R''_2$-Hal

worin Hal für ein Halogenatom steht und $R''_2$ einen Rest $COalc_1$ oder einen Rest $-(CH_2)_2N(alc_2)_2$ bedeutet, wobei $alc_1$ und $alc_2$ wie vorstehend definiert sind, unterzieht, um zu der entsprechenden Verbindung der Formel ($I_B$)

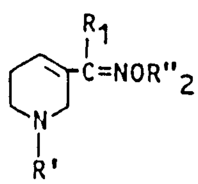

($I_B$)

zu gelangen, worin $R_1$, R' und $R''_2$ wie vorstehend definiert sind, die man gegebenenfalls entweder in ein Salz überführt oder, wenn R' für einen Aralkylrest steht, die Verbindung der Formel ($I_A$) oder ($I_B$) der Einwirkung eines Abspaltungsmittels unterzieht, um die Verbindung der Formel ($I_C$)

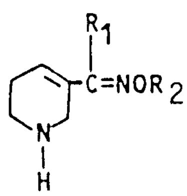

($I_C$)

zu erhalten, worin $R_1$ und $R_2$ wie vorstehend definiert sind, die man gegebenenfalls in ein Salz überführt.

**11.** Abänderung des Verfahrens gemäß Anspruch 10, dadurch gekennzeichnet, daß man eine Verbindung der Formel ($I'_A$)

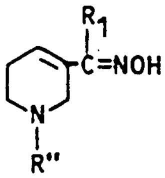

(I'$_A$)

worin R" einen Aralkylrest mit bis zu 10 Kohlenstoffatomen bedeutet und $R_1$ die vorstehend angegebene Bedeutung besitzt, der Einwirkung eines Silylierungsmittels unterzieht, um die Verbindung der Formel (VII)

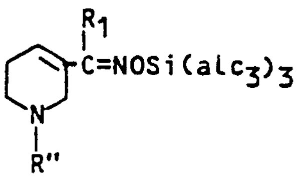

(VII)

zu erhalten, worin $alc_3$ für einen Alkylrest mit 1 bis 8 Kohlenstoffatomen steht, die man der Einwirkung eines Abspaltungsmittels unterzieht, um die Verbindung der Formel ($I_E$)

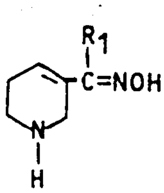

($I_E$)

zu erhalten, worin $R_1$ wie vorstehend definiert ist, welche man gegebenenfalls in ein Salz überführt.

**12.** Verfahren zur Herstellung der Verbindungen der Formel (I), worin $R_1$ und $R_2$ die vorstehend angegebene Bedeutung besitzen und R für einen Hydroxylrest steht, dadurch gekennzeichnet, daß man eine Verbindung der Formel (VIII)

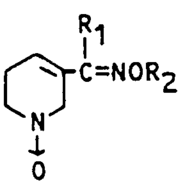

(VIII)

der Einwirkung eines Reduktionsmittels unterzieht, um zu einer Verbindung der Formel ($I_F$)

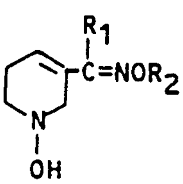

($I_F$)

zu gelangen, worin $R_1$ und $R_2$ wie vorstehend definiert sind, die man gegebenenfalls in ein Salz überführt.

13. Als Arzneimittel die Verbindungen der Formel (I) gemäß einem der Ansprüche 1 bis 9 sowie deren Additionssalze mit pharmazeutisch verträglichen Säuren.

14. Pharmazeutische Zusammensetzungen, enthaltend als Wirkstoff zumindest ein Arzneimittel gemäß Anspruch 13.

**Patentansprüche für folgenden Vertragsstaat : ES**

1. Verfahren zur Herstellung der Verbindungen der Formel (I)

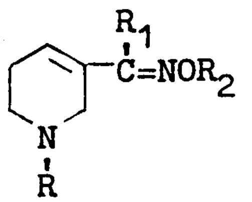

(I)

worin R ein Wasserstoffatom, einen Hydroxylrest, einen gesättigten oder ungesättigten, linearen, verzweigten oder cyclischen Alkylrest mit bis zu 8 Kohlenstoffatomen wiedergibt oder R einen Aralkylrest mit bis zu 10 Kohlenstoffatomen bedeutet, $R_1$ für einen gesättigten oder ungesättigten, linearen, verzweigten oder cyclischen Alkylrest mit bis zu 8 Kohlenstoffatomen steht und $R_2$ ein Wasserstoffatom oder einen gesättigten oder ungesättigten, linearen oder verzweigten Alkylrest mit bis zu 8 Kohlenstoffatomen, einen Rest $COalc_1$ oder einen Rest $(CH_2)_2N(alc_2)_2$ bedeutet, wobei $alc_1$ und $alc_2$ einen Alkylrest mit bis zu 8 Kohlenstoffatomen wiedergeben, sowie von deren Additionssalzen mit Säuren, mit der Maßgabe, daß, wenn R für einen Alkylrest steht, $R_2$ kein Wasserstoffatom bedeutet, dadurch gekennzeichnet, daß man

(a) zur Herstellung der Verbindungen der Formel (I), worin $R_1$ und $R_2$ die vorstehend angegebene Bedeutung besitzen und R die vorstehend angegebene Bedeutung mit Ausnahme von Hydroxyl besitzt, eine Verbindung der Formel (II)

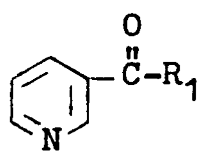

(II)

worin $R_1$ die vorstehend angegebene Bedeutung besitzt, der Einwirkung einer Verbindung der Formel (III)

$NH_2OR'_2$ (III)

oder derjenigen eines ihrer Salze, worin $R'_2$ ein Wasserstoffatom oder einen gesättigten oder ungesättigten, linearen oder verzweigten Alkylrest mit bis zu 8 Kohlenstoffatomen bedeutet, unterzieht, um die Verbindung der Formel (IV)

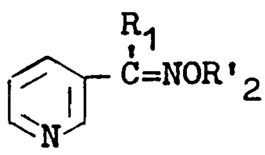

(IV)

zu erhalten, die man der Einwirkung eines Alkylhalogenids der Formel (V)

R'-Hal (V)

worin Hal für ein Halogenatom steht und R' einen gesättig ten oder ungesättigten, linearen, verzweigten oder cyclischen Alkylrest mit bis zu 8 Kohlenstoffatomen, der gegebenenfalls durch eine freie oder veresterte Carboxygruppe substituiert ist, bedeutet oder R' einen Aralkylrest mit bis zu 10 Kohlenstoffatomen bedeutet, unterzieht, um die Verbindung der Formel (VI)

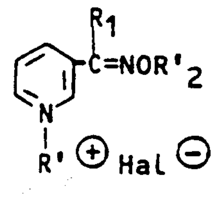

(VI)

zu erhalten, die man der Einwirkung eines Hydrierungsmittels unterzieht, um zu der Verbindung der Formel ($I_A$)

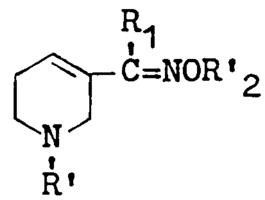

($I_A$)

worin R', $R_1$ und $R'_2$ wie vorstehend definiert sind, zu gelangen, die man gegebenenfalls entweder in ein Salz überführt oder, wenn $R'_2$ für ein Wasserstoffatom steht, der Einwirkung einer Verbindung der Formel

$R''_2$-Hal

worin Hal für ein Halogenatom steht und $R''_2$ einen Rest $COalc_1$ oder einen Rest $-(CH_2)_2N(alc_2)_2$ bedeutet, wobei $alc_1$ und $alc_2$ wie vorstehend definiert sind, unterzieht, um zu der entsprechenden Verbindung der Formel ($I_B$)

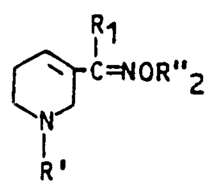

($I_B$)

zu gelangen, worin $R_1$, R' und $R''_2$ wie vorstehend definiert sind, die man gegebenenfalls in ein Salz überführt oder, wenn R' einen Aralkylrest bedeutet, die Verbindung der Formel ($I_A$) oder ($I_B$) der Einwirkung eines Abspaltungsmittels unterzieht, um zu der Verbindung der Formel ($I_C$)

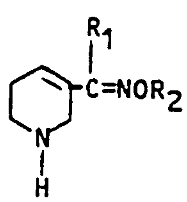

($I_C$)

worin $R_1$ und $R_2$ wie vorstehend definiert sind, zu gelangen, die man gegebenenfalls in ein Salz überführt;
(b) zur Herstellung der Verbindungen der Formel (I), worin $R_1$ und $R_2$ die vorstehend angegebene

Bedeutung besitzen und R einen Hydroxylrest wiedergibt, eine Verbindung der Formel (VIII)

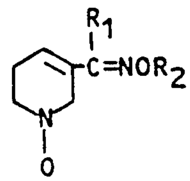

(VIII)

der Einwirkung eines Reduktionsmittels unterzieht, um eine Verbindung der Formel ($I_F$)

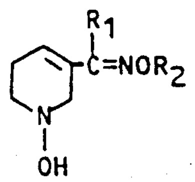

($I_F$)

worin $R_1$ und $R_2$ wie vorstehend definiert sind, zu erhalten, die man gegebenenfalls in ein Salz überführt.

**2.** Verfahren gemäß Anspruch 1 zur Herstellung der Verbindungen der Formel (I), worin $R_1$ und $R_2$ die vorstehend angegebene Bedeutung besitzen und R die vorstehend angegebene Bedeutung mit Ausnahme von Hydroxyl aufweist, dadurch gekennzeichnet, daß man eine Verbindung der Formel (II)

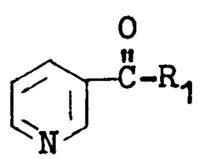

(II)

worin $R_1$ die vorstehend angegebene Bedeutung besitzt, der Einwirkung einer Verbindung der Formel (III)

$NH_2OR'_2$ (III)

oder derjenigen eines ihrer Salze, worin $R'_2$ ein Wasserstoffatom oder einen gesättigten oder ungesättigten, linearen oder verzweigten Alkylrest mit bis zu 8 Kohlenstoffatomen bedeutet, unterzieht, um zu der Verbindung der Formel (IV)

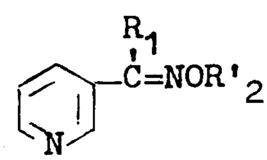

(IV)

zu gelangen, die man der Einwirkung eines Alkylhalogenids der Formel (V)

R'-Hal (V)

unterzieht, worin Hal für ein Halogenatom steht und R' einen gesättigten oder ungesättigten, linearen, verzweigten oder cyclischen Alkylrest mit bis zu 8 Kohlenstoffatomen, der gegebenenfalls durch eine freie oder veresterte Carboxygruppe substituiert ist, bedeutet oder R' für einen Aralkylrest mit bis zu 10 Kohlenstoffatomen steht, um zu der Verbindung der Formel (VI)

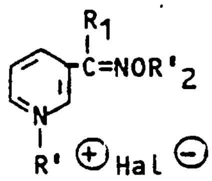

(VI)

zu gelangen, die man der Einwirkung eines Hydrierungsmittels unterzieht, um die Verbindung der Formel ($I_A$)

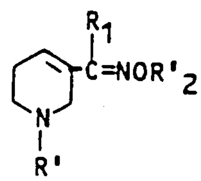

($I_A$)

worin R', $R_1$ und $R'_2$ wie vorstehend definiert sind, zu erhalten, die man gegebenenfalls entweder in ein Salz überführt oder, wenn $R'_2$ ein Wasserstoffatom bedeutet, der Einwirkung einer Verbindung der Formel

$R''_2$-Hal

worin Hal für ein Halogenatom steht und $R''_2$ einen Rest $COalc_1$ oder einen Rest $-(CH_2)_2N(alc_2)_2$ bedeutet, wobei $alc_1$ und $alc_2$ wie vorstehend definiert sind, unterzieht, um zu der entsprechenden Verbindung der Formel ($I_B$)

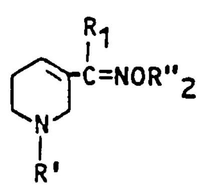

($I_B$)

zu gelangen, worin $R_1$, R' und $R''_2$ wie vorstehend definiert sind, die man gegebenenfalls entweder in ein Salz überführt oder, wenn R' für einen Aralkylrest steht, der Einwirkung eines Abspaltungsmittels unterzieht, um die Verbindung der Formel ($I_C$)

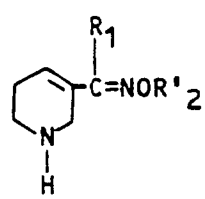

($I_C$)

zu erhalten, worin $R_1$ und $R'_2$ wie vorstehend definiert sind, die man gegebenenfalls in ein Salz überführt.

**3.** Verfahren gemäß Anspruch 2 zur Herstellung der Verbindungen der Formel (I), wie in Anspruch 1 definiert, der Formel ($I_A$)

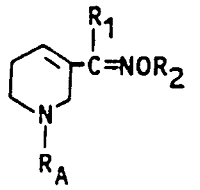

$(I_A)$

worin $R_1$ und $R_2$ die in Anspruch 1 angegebene Bedeutung besitzen und $R_A$ für ein Wasserstoffatom, einen gesättigten oder ungesättigten, linearen, verzweigten oder cyclischen Alkylrest mit bis zu 8 Kohlenstoffatomen, der gegebenenfalls durch eine freie oder veresterte Carboxygruppe substituiert ist, steht oder R einen Aralkylrest mit bis zu 10 Kohlenstoffatomen bedeutet, sowie von deren Additionssalzen mit Säuren, mit der Maßgabe, daß, wenn $R_A$ für einen Alkylrest steht, $R_2$ kein Wasserstoffatom bedeutet, dadurch gekennzeichnet, daß man eine Verbindung der Formel (II)

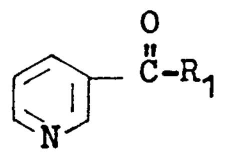

(II)

worin $R_1$ die vorstehend angegebene Bedeutung besitzt, der Einwirkung einer Verbindung der Formel (III)

$NH_2OR'_2$ (III)

oder derjenigen eines ihrer Salze, worin $R'_2$ für ein Wasserstoffatom oder einen gesättigten oder ungesättigten, linearen oder verzweigten Alkylrest mit bis zu 8 Kohlenstoffatomen steht, unterzieht, um die Verbindung der Formel (IV)

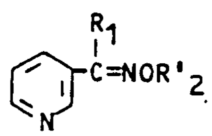

(IV)

zu erhalten, die man der Einwirkung eines Alkylhalogenids der Formel (V)

R'-Hal (V)

unterzieht, worin Hal für ein Halogenatom steht und R' einen gesättigten oder ungesättigten, linearen, verzweigten oder cyclischen Alkylrest mit bis zu 8 Kohlenstoffatomen bedeutet, der gegebenenfalls durch eine freie oder veresterte Carboxygruppe substituiert ist, oder R' einen Aralkylrest mit bis zu 10 Kohlenstoffatomen bedeutet, um zu der Verbindung der Formel (VI)

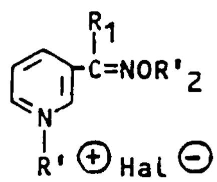

(VI)

zu gelangen, die man der Einwirkung eines Hydrierungsmittels unterzieht, um die Verbindung der Formel $(I_A)$

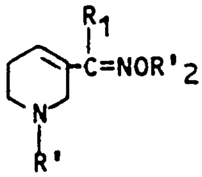

$(I_A)$

zu erhalten, worin R', $R_1$ und $R'_2$ wie vorstehend definiert sind, die man gegebenenfalls entweder in ein Salz überführt oder, wenn $R'_2$ für ein Wasserstoffatom steht, der Einwirkung einer Verbindung der Formel

$R''_2$-Hal

worin Hal für ein Halogenatom steht und $R''_2$ einen Rest $COalc_1$ oder einen Rest $-(CH_2)_2N(alc_2)_2$ bedeutet, wobei $alc_1$ und $alc_2$ wie vorstehend definiert sind, unterzieht, um zu der entsprechenden Verbindung der Formel $(I_B)$

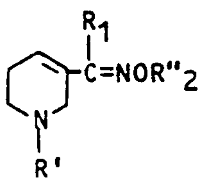

$(I_B)$

zu gelangen, worin $R_1$, R' und $R''_2$ wie vorstehend definiert sind, die man gegebenenfalls entweder in ein Salz überführt oder, wenn R' für einen Aralkylrest steht, der Einwirkung eines Abspaltungsmittels unterzieht, um zu der Verbindung der Formel $(I_C)$

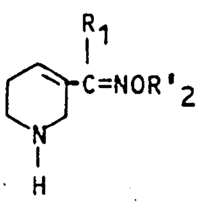

$(I_C)$

zu gelangen, worin $R_1$ und $R'_2$ wie vorstehend definiert sind, die man gegebenenfalls in ein Salz überführt.

**4.** Abänderung des Verfahrens gemaß Anspruch 3, dadurch gekennzeichnet, daß man eine Verbindung der Formel $(I'_A)$

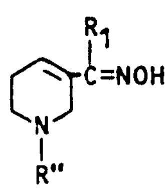

$(I'_A)$

worin R" einen Aralkylrest mit bis zu 10 Kohlenstoffatomen bedeutet und $R_1$ die vorstehend angegebene Bedeutung besitzt, der Einwirkung eines Silylierungsmittels unterzieht, um die Verbindung der Formel (VII)

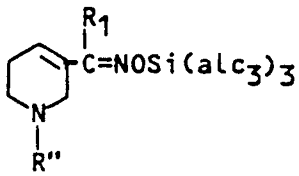

(VII)

zu erhalten, worin $alc_3$ für einen Alkylrest mit 1 bis 8 Kohlenstoffatomen steht, die man der Einwirkung eines Abspaltungsmittels unterzieht, um die Verbindung der Formel ($I_E$)

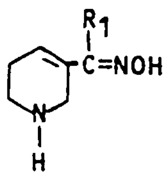

($I_E$)

zu erhalten, worin $R_1$ wie vorstehend definiert ist, welche man gegebenenfalls in ein Salz überführt.

5. Verfahren gemäß Anspruch 1 zur Herstellung der Verbindungen der Formel (I), worin $R_1$ und $R_2$ die vorstehend angegebene Bedeutung besitzen und R einen Hydroxylrest wiedergibt, dadurch gekennzeichnet, daß man eine Verbindung der Formel (VIII)

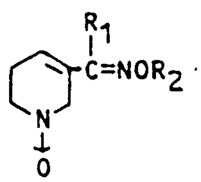

(VIII)

der Einwirkung eines Reduktionsmittels unterzieht, um zu einer Verbindung der Formel ($I_F$)

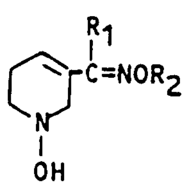

($I_F$)

zu gelangen, worin $R_1$ und $R_2$ wie vorstehend definiert sind, die man gegebenenfalls in ein Salz überführt.

6. Verfahren gemäß einem der Ansprüche 1 bis 5, dadurch gekennzeichnet, daß man von einer Verbindung ausgeht, in der $R_1$ für einen linearen Alkylrest mit 1 bis 4 Kohlenstoffatomen steht.

7. Verfahren gemäß einem der Ansprüche 1 bis 5, dadurch gekennzeichnet, daß man von einer Verbindung ausgeht, in der $R_1$ für einen Methylrest steht.

8. Verfahren gemäß Anspruch 3, dadurch gekennzeichnet, daß man von einer Verbindung der Formel (V) ausgeht, worin R' für einen gesättigten oder ungesättigten, linearen oder verzweigten Alkylrest mit 1 bis 4 Kohlenstoffatomen steht.

9. Verfahren gemäß Anspruch 3, dadurch gekennzeichnet, daß man von einer Verbindung der Formel (V) ausgeht, worin R' einen Methyl-, Ethyl-, Propyl- oder Allylrest bedeutet.

**10.** Verfahren gemäß einem der Ansprüche 1 bis 3 und 5 bis 9, dadurch gekennzeichnet, daß man von einer Verbindung ausgeht, worin $R'_2$ oder $R_2$ ein Wasserstoffatom bedeutet.

**11.** Verfahren gemäß einem der Ansprüche 1 bis 3 und 5 bis 9, dadurch gekennzeichnet, daß man von einer Verbindung ausgeht, worin $R'_2$ oder $R_2$ einen Alkylrest mit 1 bis 4 Kohlenstoffatomen bedeutet.

**12.** Verfahren gemäß einem der Ansprüche 1 bis 3 und 5 bis 9, dadurch gekennzeichnet, daß man von einer Verbindung ausgeht, worin $R'_2$ oder $R_2$ einen Methylrest bedeutet.

**13.** Verfahren gemäß Anspruch 1, dadurch gekennzeichnet, daß man Verbindungen der Formel (I), worin R ein Wasserstoffatom oder eine Hydroxylgruppe darstellt, sowie deren Additionssalze mit Säuren herstellt.

**14.** Verfahren gemäß Anspruch 1, dadurch gekennzeichnet, daß man irgendeine der Verbindungen der Formel (I) mit den folgenden Bezeichnungen herstellt:
1-Methyl-3-acetyl-1,2,5,6-tetrahydropyridin-O-methyloxim und dessen Hydrochlorid,
3-Acetyl-1,2,5,6-tetrahydropyridin-O-methyloxim und dessen Hydrochlorid,
1-N-Hydroxyl-3-acetyl-1,2,5,6-tetrahydropyridin-O-methyloxim.

**Patentansprüche für folgenden Vertragsstaat : GR**

**1.** Verfahren zur Herstellung der Verbindungen der Formel (I)

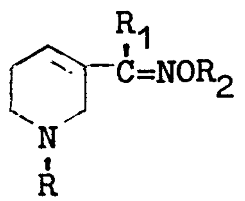

(I)

worin R ein Wasserstoffatom, einen Hydroxylrest, einen gesättigten oder ungesättigten, linearen, verzweigten oder cyclischen Alkylrest mit bis zu 8 Kohlenstoffatomen wiedergibt oder R einen Aralkylrest mit bis zu 10 Kohlenstoffatomen bedeutet, $R_1$ für einen gesättigten oder ungesättigten, linearen, verzweigten oder cyclischen Alkylrest mit bis zu 8 Kohlenstoffatomen steht und $R_2$ ein Wasserstoffatom oder einen gesättigten oder ungesättigten, linearen oder verzweigten Alkylrest mit bis zu 8 Kohlenstoffatomen, einen Rest $COalc_1$ oder einen Rest $(CH_2)_2N(alc_2)_2$ bedeutet, wobei $alc_1$ und $alc_2$ einen Alkylrest mit bis zu 8 Kohlenstoffatomen wiedergeben, sowie von deren Additionssalzen mit Säuren, mit der Maßgabe, daß, wenn R für einen Alkylrest steht, $R_2$ kein Wasserstoffatom bedeutet,
dadurch gekennzeichnet, daß man

(a) zur Herstellung der Verbindungen der Formel (I), worin $R_1$ und $R_2$ die vorstehend angegebene Bedeutung besitzen und R die vorstehend angegebene Bedeutung mit Ausnahme von Hydroxyl besitzt, eine Verbindung der Formel (II)

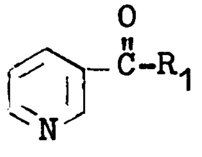

(II)

worin $R_1$ die vorstehend angegebene Bedeutung besitzt, der Einwirkung einer Verbindung der Formel (III)

$NH_2OR'_2$ (III)

oder derjenigen eines ihrer Salze, worin $R'_2$ ein Wasserstoffatom oder einen gesättigten oder ungesättigten, linearen oder verzweigten Alkylrest mit bis zu 8 Kohlenstoffatomen bedeutet, unterzieht, um die Verbindung der Formel (IV)

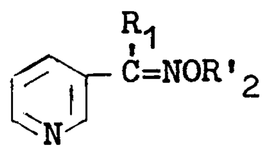

(IV)

zu erhalten, die man der Einwirkung eines Alkylhalogenids der Formel (V)

R'-Hal (V)

worin Hal für ein Halogenatom steht und R' einen gesättigten oder ungesättigten, linearen, verzweigten oder cyclischen Alkylrest mit bis zu 8 Kohlenstoffatomen, der gegebenenfalls durch eine freie oder veresterte Carboxygruppe substituiert ist, bedeutet oder R' einen Aralkylrest mit bis zu 10 Kohlenstoffatomen bedeutet, unterzieht, um die Verbindung der Formel (VI)

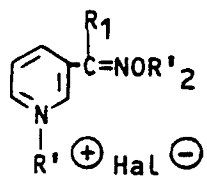

(VI)

zu erhalten, die man der Einwirkung eines Hydrierungsmittels unterzieht, um zu der Verbindung der Formel ($I_A$)

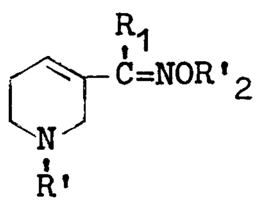

($I_A$)

worin R', $R_1$ und $R'_2$ wie vorstehend definiert sind, zu gelangen, die man gegebenenfalls entweder in ein Salz überführt oder, wenn $R'_2$ für ein Wasserstoffatom steht, der Einwirkung einer Verbindung der Formel

$R''_2$-Hal

worin Hal für ein Halogenatom steht und $R''_2$ einen Rest $COalc_1$ oder einen Rest $-(CH_2)_2N(alc_2)_2$ bedeutet, wobei $alc_1$ und $alc_2$ wie vorstehend definiert sind, unterzieht, um zu der entsprechenden Verbindung der Formel ($I_B$)

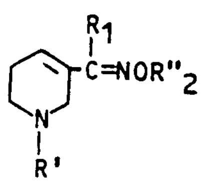

($I_B$)

zu gelangen, worin $R_1$, R' und $R''_2$ wie vorstehend definiert sind, die man gegebenenfalls in ein Salz überführt oder, wenn R' einen Aralkylrest bedeutet, die Verbindung der Formel ($I_A$) oder ($I_B$) der

Einwirkung eines Abspaltungsmittels unterzieht, um zu der Verbindung der Formel ($I_C$)

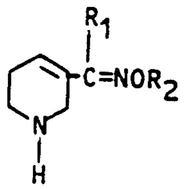

($I_C$)

worin $R_1$ und $R_2$ wie vorstehend definiert sind, zu gelangen, die man gegebenenfalls in ein Salz überführt;

(b) zur Herstellung der Verbindungen der Formel (I), worin $R_1$ und $R_2$ die vorstehend angegebene Bedeutung besitzen und R einen Hydroxylrest wiedergibt, eine Verbindung der Formel (VIII)

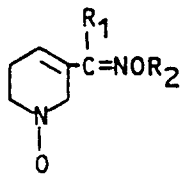

(VIII)

der Einwirkung eines Reduktionsmittels unterzieht, um eine Verbindung der Formel ($I_F$)

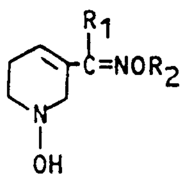

($I_F$)

worin $R_1$ und $R_2$ wie vorstehend definiert sind, zu erhalten, die man gegebenenfalls in ein Salz überführt.

**2.** Verfahren gemäß Anspruch 1 zur Herstellung der Verbindungen der Formel (I), worin $R_1$ und $R_2$ die vorstehend angegebene Bedeutung besitzen und R die vorstehend angegebene Bedeutung mit Ausnahme von Hydroxyl aufweist, dadurch gekennzeichnet, daß man eine Verbindung der Formel (II)

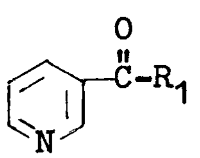

(II)

worin $R_1$ die vorstehend angegebene Bedeutung besitzt, der Einwirkung einer Verbindung der Formel (III)

$NH_2OR'_2$ (III)

oder derjenigen eines ihrer Salze, worin $R'_2$ ein Wasserstoffatom oder einen gesättigten oder ungesättigten, linearen oder verzweigten Alkylrest mit bis zu 8 Kohlenstoffatomen bedeutet, unterzieht, um zu der Verbindung der Formel (IV)

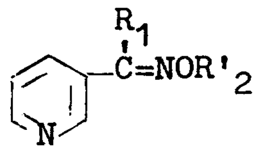

(IV)

zu gelangen, die man der Einwirkung eines Alkylhalogenids der Formel (V)

R'-Hal (V)

unterzieht, worin Hal für ein Halogenatom steht und R' einen gesättigten oder ungesättigten, linearen, verzweigten oder cyclischen Alkylrest mit bis zu 8 Kohlenstoffatomen, der gegebenenfalls durch eine freie oder veresterte Carboxygruppe substituiert ist, bedeutet oder R' für einen Aralkylrest mit bis zu 10 Kohlenstoffatomen steht, um zu der Verbindung der Formel (VI)

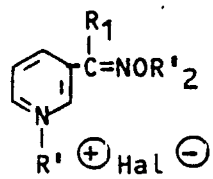

(VI)

zu gelangen, die man der Einwirkung eines Hydrierungsmittels unterzieht, um die Verbindung der Formel ($I_A$)

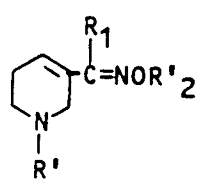

($I_A$)

worin R', $R_1$ und $R'_2$ wie vorstehend definiert sind, zu erhalten, die man gegebenenfalls entweder in ein Salz überführt oder, wenn $R'_2$ ein Wasserstoffatom bedeutet, der Einwirkung einer Verbindung der Formel

$R''_2$-Hal

worin Hal für ein Halogenatom steht und $R''_2$ einen Rest $COalc_1$ oder einen Rest $-(CH_2)_2N(alc_2)_2$ bedeutet, wobei $alc_1$ und $alc_2$ wie vorstehend definiert sind, unterzieht, um zu der entsprechenden Verbindung der Formel ($I_B$)

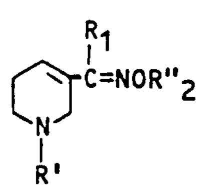

($I_B$)

zu gelangen, worin $R_1$, R' und $R''_2$ wie vorstehend definiert sind, die man gegebenenfalls entweder in ein Salz überführt oder, wenn R' für einen Aralkylrest steht, der Einwirkung eines Abspaltungsmittels unterzieht, um die Verbindung der Formel ($I_C$)

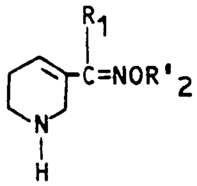

$(I_C)$

zu erhalten, worin $R_1$ und $R'_2$ wie vorstehend definiert sind, die man gegebenenfalls in ein Salz überführt.

**3.** Verfahren gemäß Anspruch 2 zur Herstellung der Verbindungen der Formel (I), wie in Anspruch 1 definiert, der Formel $(I_A)$

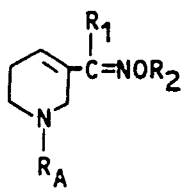

$(I_A)$

worin $R_1$ und $R_2$ die in Anspruch 1 angegebene Bedeutung besitzen und $R_A$ für ein Wasserstoffatom, einen gesättigten oder ungesättigten, linearen, verzweigten oder cyclischen Alkylrest mit bis zu 8 Kohlenstoffatomen, der gegebenenfalls durch eine freie oder veresterte Carboxygruppe substituiert ist, steht oder R einen Aralkylrest mit bis zu 10 Kohlenstoffatomen bedeutet, sowie von deren Additionssalzen mit Säuren, mit der Maßgabe, daß, wenn $R_A$ für einen Alkylrest steht, $R_2$ kein Wasserstoffatom bedeutet, dadurch gekennzeichnet, daß man eine Verbindung der Formel (II)

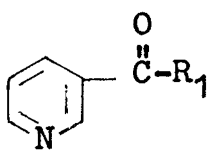

(II)

worin $R_1$ die vorstehend angegebene Bedeutung besitzt, der Einwirkung einer Verbindung der Formel (III)

$NH_2OR'_2$ (III)

oder derjenigen eines ihrer Salze, worin $R'_2$ für ein Wasserstoffatom oder einen gesättigten oder ungesättigten, linearen oder verzweigten Alkylrest mit bis zu 8 Kohlenstoffatomen steht, unterzieht, um die Verbindung der Formel (IV)

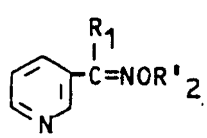

(IV)

zu erhalten, die man der Einwirkung eines Alkylhalogenids der Formel (V)

R'-Hal (V)

unterzieht, worin Hal für ein Halogenatom steht und R' einen gesättigten oder ungesättigten, linearen, verzweigten oder cyclischen Alkylrest mit bis zu 8 Kohlenstoffatomen bedeutet, der gegebenenfalls durch eine freie oder veresterte Carboxygruppe substituiert ist, oder R' einen Aralkylrest mit bis zu 10

Kohlenstoffatomen bedeutet, um zu der Verbindung der Formel (VI)

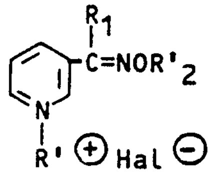

(VI)

zu gelangen, die man der Einwirkung eines Hydrierungsmittels unterzieht, um die Verbindung der Formel ($I_A$)

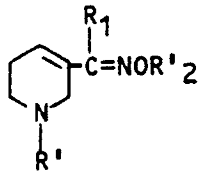

($I_A$)

zu erhalten, worin R', $R_1$ und $R'_2$ wie vorstehend definiert sind, die man gegebenenfalls entweder in ein Salz überführt oder, wenn $R'_2$ für ein Wasserstoffatom steht, der Einwirkung einer Verbindung der Formel

$R''_2$-Hal

worin Hal für ein Halogenatom steht und $R''_2$ einen Rest $COalc_1$ oder einen Rest $-(CH_2)_2N(alc_2)_2$ bedeutet, wobei $alc_1$ und $alc_2$ wie vorstehend definiert sind, unterzieht, um zu der entsprechenden Verbindung der Formel ($I_B$)

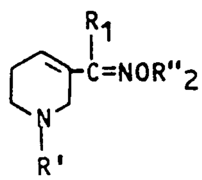

($I_B$)

zu gelangen, worin $R_1$, R' und $R''_2$ wie vorstehend definiert sind, die man gegebenenfalls entweder in ein Salz überführt oder, wenn R' für einen Aralkylrest steht, der Einwirkung eines Abspaltungsmittels unterzieht, um zu der Verbindung der Formel ($I_C$)

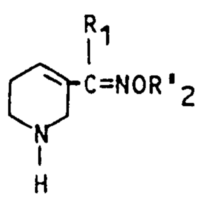

($I_C$)

zu gelangen, worin $R_1$ und $R'_2$ wie vorstehend definiert sind, die man gegebenenfalls in ein Salz überführt.

**4.** Abänderung des Verfahrens gemäß Anspruch 3, dadurch gekennzeichnet, daß man eine Verbindung der Formel ($I'_A$)

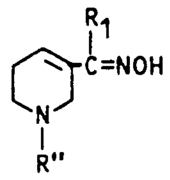

(I'$_A$)

worin R" einen Aralkylrest mit bis zu 10 Kohlenstoffatomen bedeutet und $R_1$ die vorstehend angegebene Bedeutung besitzt, der Einwirkung eines Silylierungsmittels unterzieht, um die Verbindung der Formel (VII)

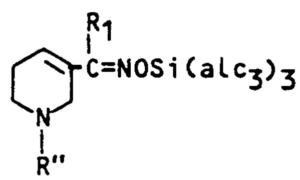

(VII)

zu erhalten, worin $alc_3$ für einen Alkylrest mit 1 bis 8 Kohlenstoffatomen steht, die man der Einwirkung eines Abspaltungsmittels unterzieht, um die Verbindung der Formel ($I_E$)

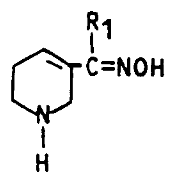

($I_E$)

zu erhalten, worin $R_1$ wie vorstehend definiert ist, welche man gegebenenfalls in ein Salz überführt.

**5.** Verfahren gemäß Anspruch 1 zur Herstellung der Verbindungen der Formel (I), worin $R_1$ und $R_2$ die vorstehend angegebene Bedeutung besitzen und R einen Hydroxylrest wiedergibt, dadurch gekennzeichnet, daß man eine Verbindung der Formel (VIII)

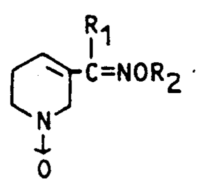

(VIII)

der Einwirkung eines Reduktionsmittels unterzieht, um zu einer Verbindung der Formel ($I_F$)

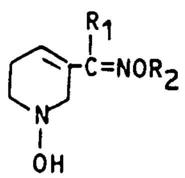

($I_F$)

zu gelangen, worin $R_1$ und $R_2$ wie vorstehend definiert sind, die man gegebenenfalls in ein Salz überführt.

6. Verfahren gemäß einem der Ansprüche 1 bis 5, dadurch gekennzeichnet, daß man von einer Verbindung ausgeht, in der $R_1$ für einen linearen Alkylrest mit 1 bis 4 Kohlenstoffatomen steht.

7. Verfahren gemäß einem der Ansprüche 1 bis 5, dadurch gekennzeichnet, daß man von einer Verbindung ausgeht, in der $R_1$ für einen Methylrest steht.

8. Verfahren gemäß Anspruch 3, dadurch gekennzeichnet, daß man von einer Verbindung der Formel (V) ausgeht, worin R' für einen gesättigten oder ungesättigten, linearen oder verzweigten Alkylrest mit 1 bis 4 Kohlenstoffatomen steht.

9. Verfahren gemäß Anspruch 3, dadurch gekennzeichnet, daß man von einer Verbindung der Formel (V) ausgeht, worin R' einen Methyl-, Ethyl-, Propyl- oder Allylrest bedeutet.

10. Verfahren gemäß einem der Ansprüche 1 bis 3 und 5 bis 9, dadurch gekennzeichnet, daß man von einer Verbindung ausgeht, worin $R'_2$ oder $R_2$ ein Wasserstoffatom bedeutet.

11. Verfahren gemäß einem der Ansprüche 1 bis 3 und 5 bis 9, dadurch gekennzeichnet, daß man von einer Verbindung ausgeht, worin $R'_2$ oder $R_2$ einen Alkylrest mit 1 bis 4 Kohlenstoffatomen bedeutet.

12. Verfahren gemäß einem der Ansprüche 1 bis 3 und 5 bis 9, dadurch gekennzeichnet, daß man von einer Verbindung ausgeht, worin $R'_2$ oder $R_2$ einen Methylrest bedeutet.

13. Verfahren gemaß Anspruch 1, dadurch gekennzeichnet, daß man Verbindungen der Formel (I), worin R ein Wasserstoffatom oder eine Hydroxylgruppe darstellt, sowie deren Additionssalze mit Säuren herstellt.

14. Verfahren gemäß Anspruch 1, dadurch gekennzeichnet, daß man irgendeine der Verbindungen der Formel (I) mit den folgenden Bezeichnungen herstellt:
    1-Methyl-3-acetyl-1,2,5,6-tetrahydropyridin-O-methyloxim und dessen Hydrochlorid,
    3-Acetyl-1,2,5,6-tetrahydropyridin-O-methyloxim und dessen Hydrochlorid,
    1-N-Hydroxyl-3-acetyl-1,2,5,6-tetrahydropyridin-O-methyloxim.

15. Verfahren zur Herstellung von pharmazeutischen Zusammensetzungen, dadurch gekennzeichnet, daß man als Wirkstoff zumindest eine der Verbindungen der Formel (I), wie in Anspruch 1 definiert, oder zumindest eines ihrer Additionssalze mit pharmazeutisch verträglichen Säuren in eine für diese Verwendung bestimmte Form überführt.

16. Verfahren zur Herstellung von pharmazeutischen Zusammensetzungen, dadurch gekennzeichnet, daß man als Wirkstoff zumindest eine der Verbindungen der Formel ($I_A$), wie in Anspruch 3 definiert, oder zumindest eines ihrer Additionssalze mit pharmazeutisch verträglichen Säuren in eine für diese Verwendung bestimmte Form überführt.

17. Verfahren zur Herstellung von pharmazeutischen Zusammensetzungen, dadurch gekennzeichnet, daß man als Wirkstoff zumindest eine der Verbindungen der Formel (I), wie in Anspruch 14 definiert, oder zumindest eines ihrer Additionssalze mit pharmazeutisch verträglichen Säuren in eine für diese Verwendung bestimmte Form überführt.